(19) Europäisches Patentamt
European Patent Office
Office européen des brevets

(11) Veröffentlichungsnummer: **0 092 112**
**B1**

(12) # EUROPÄISCHE PATENTSCHRIFT

(45) Veröffentlichungstag der Patentschrift: **06.03.85**

(21) Anmeldenummer: **83103373.3**

(22) Anmeldetag: **07.04.83**

(51) Int. Cl.⁴: **C 07 C 149/14,** C 07 C 147/02, C 07 C 147/14, C 07 D 213/64, A 01 N 31/04, A 01 N 43/40

(54) **Substituierte Phenoxypropionate.**

(30) Priorität: **20.04.82 JP 64702/82**

(43) Veröffentlichungstag der Anmeldung: **26.10.83 Patentblatt 83/43**

(45) Bekanntmachung des Hinweises auf die Patenterteilung: **06.03.85 Patentblatt 85/10**

(84) Benannte Vertragsstaaten: **AT BE CH DE FR GB IT LI NL SE**

(56) Entgegenhaltungen:
**EP - A - 0 030 702**
**DE - A - 2 617 804**
**DE - A - 2 623 558**
**DE - A - 2 812 571**

(73) Patentinhaber: **NIHON TOKUSHU NOYAKU SEIZO K.K., No.4, 2-Chome, Nihonbashi Honcho, Chuo-ku Tokyo 103 (JP)**

(72) Erfinder: **Saito, Junichi, 3-7-12, Osawa, Mitaka-shi Tokyo (JP)**
Erfinder: **Yasui, Kazuomi, 7-6-15, Shakujii-dai, Nerima-ku Tokyo (JP)**
Erfinder: **Shiokawa, Kozo, 210-6, Shukugawara Tama-ku, Kawasaki-shi Kanagawa-ken (JP)**
Erfinder: **Kamochi, Atsumi, 2-24-10, Higashi-Toyoda, Hino-shi Tokyo (JP)**

(74) Vertreter: **Schumacher, Günter, Dr. et al, c/o Bayer AG Konzernverwaltung RP Patentabteilung, D-5090 Leverkusen 1 Bayerwerk (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 092 112 B1

**Beschreibung**

Die vorliegende Erfindung betrifft neue substituierte Phenoxypropionsäureester, Zwischenprodukte zu deren Herstellung, Verfahren zur Herstellung dieser Ester und der Zwischenprodukte und sie enthaltende Herbizide.

Die vorliegende Erfindung betrifft insbesondere substituierte Phenoxypropionate der nachstehenden allgemeinen Formel (I)

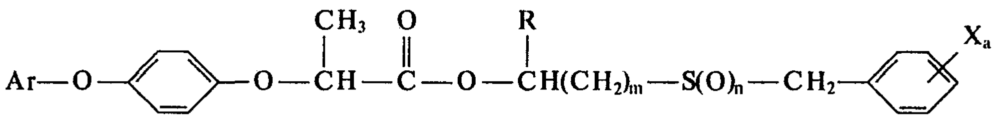

in der

Ar die Gruppe 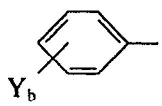 oder die Gruppe 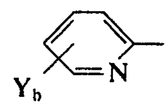 bezeichnet,

in der Y für ein Halogen-Atom oder eine Trifluoromethyl-Gruppe und b für 1 oder 2 stehen,

R ein Wasserstoff-Atom oder eine Methyl-Gruppe bezeichnet,

X ein Wasserstoff-Atom, ein Halogen-Atom, eine Nitro-Gruppe, eine niedere Alkyl-Gruppe oder eine niedere Alkoxy-Gruppe bezeichnet,

a und m jeweils für 1 oder 2 stehen und

n für 0, 1 oder 2 steht.

Die substituierten Phenoxypropionate der allgemeinen Formel (I) können beispielsweise mittels der folgenden Verfahren i), ii), iii) und iv) hergestellt werden, auf die sich die vorliegende Erfindung ebenfalls bezieht.

Verfahren i)

Ein Verfahren zur Herstellung der substituierten Phenoxypropionate der allgemeinen Formel (I) ist dadurch gekennzeichnet, daß eine Verbindung der allgemeinen Formel (II)

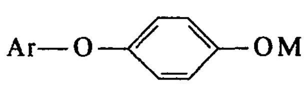 (II)

in der

Ar die im Vorstehenden angegebene Bedeutung hat und

M ein Wasserstoff-Atom oder ein Alkylimetall-Atom bezeichnet,

mit einer Verbindung der allgemeinen Formel (III)

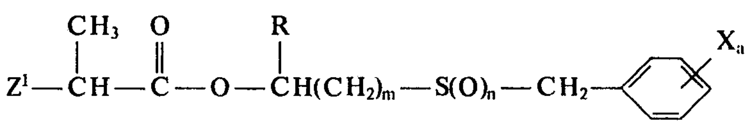 (III)

in der

R, X, a, m und n die im Vorstehenden angegebenen Bedeutungen haben und

$Z^1$ für ein Halogen-Atom steht,

zur Reaktion gebracht wird.

Verfahren ii)

Ein Verfahren zur Herstellung der substituierten Phenoxypropionate der allgemeinen Formel (I) ist dadurch gekennzeichnet, daß eine Verbindung der allgemeinen Formel (IV)

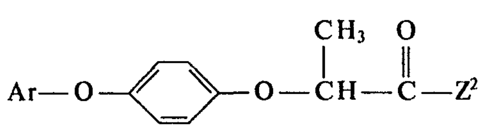 (IV)

in der

Ar die im Vorstehenden angegebene Bedeutung hat und
$Z^2$ für eine Hydroxyl-Gruppe oder ein Halogen-Atom steht,

mit einer Verbindung der allgemeinen Formel (V)

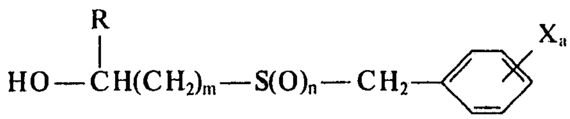
(V)

in der
R, X, a, m und n die im Vorstehenden angegebenen Bedeutungen haben,
zur Reaktion gebracht wird.

Verfahren iii)

Ein Verfahren zur Herstellung der substituierten Phenoxypropionate der allgemeinen Formel (I) ist dadurch gekennzeichnet, daß eine Verbindung der allgemeinen Formel (VI)

$$Ar—Z^1 \qquad (VI)$$

in der
Ar und $Z^1$ die im Vorstehenden angegebenen Bedeutungen haben,
mit einer Verbindung der allgemeinen Formel (VII)

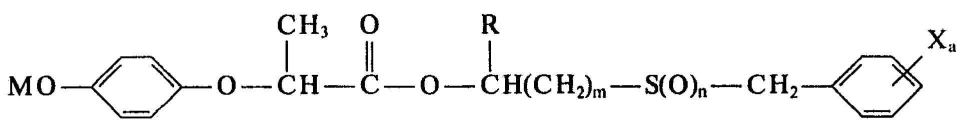
(VII)

in der
R, X, a, m, n und M die im Vorstehenden angebebenen Bedeutungen haben,
zur Reaktion gebracht wird.

Verfahren iv)

Ein Verfahren zur Herstellung der substituierten Phenoxypropionate der allgemeinen Formel (I-ii)

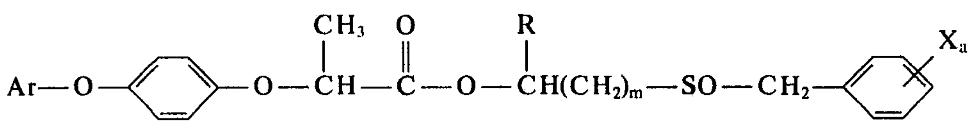
(I-ii)

in der
Ar, R, X, a und m die im Vorstehenden angegebenen Bedeutungen haben,
ist dadurch gekennzeichnet, daß eine Verbindung der allgemeinen Formel (I-i),

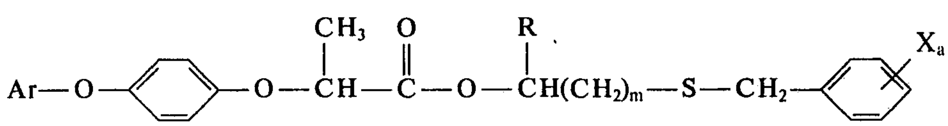
(I-i)

in der
Ar, R, X, a und m die im Vorstehenden angegebenen Bedeutungen haben,
mit Wasserstoffperoxid zur Reaktion gebracht wird.

Die vorliegende Erfindung betrifft außerdem ein Herbizid, das ein substituiertes Phenoxypropionat der allgemeinen Formel (I) als Wirkstoff enthält.

Die vorliegende Erfindung betrifft weiterhin 2-Halogenopropionate der vorstehenden allgemeinen Formel (III), die Zwischenprodukte für die Herstellung der substituierten Phenoxypropionate der allgemeinen Formel (I) sind.

Die vorliegende Erfindung betrifft weiter ein Verfahren zur Herstellung der 2-Halogenopropionate der allgemeinen Formel (III) als Zwischenprodukte [Verfahren v)].

Verfahren v)

Ein Verfahren zur Herstellung der 2-Halogenopropionate der allgemeinen Formel (III) ist dadurch

gekennzeichnet, daß eine Verbindung der allgemeinen Formel (VIII)

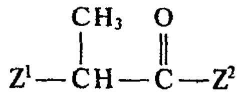

(VIII)

in der
$Z^1$ und $Z^2$ die im vorstehenden angegebenen Bedeutungen haben,
mit einer Verbindung der vorstehenden allgemeinen Formel (V) zur Reaktion gebracht wird.

Die vorliegende Erfindung betrifft weiterhin 2-(4-Hydroxyphenoxy)propionate oder deren Alkali-Salze der allgemeinen Formel (VII), die Zwischenprodukte für die Herstellung der substituierten Phenoxypropionate der allgemeinen Formel (I) sind.

Die vorliegende Erfindung betrifft außerdem ein Verfahren zur Herstellung der 2-(4-Hydroxyphenoxy)propionate und ihrer Alkali-Salze der allgemeinen Formel (VII).

Verfahren vi) (M ist Wasserstoff in der allgemeinen Formel (VII))

Ein Verfahren zur Herstellung der 2-(4-Hydroxyphenoxy)propionate der allgemeinen Formel (VII-i)

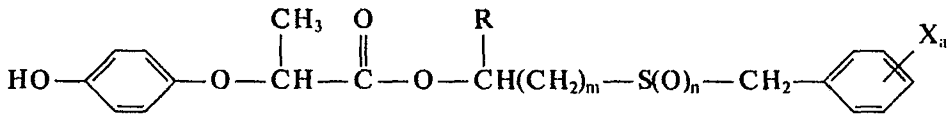

(VII-i)

in der
R, X, a, m und n die im Vorstehenden angegebenen Bedeutungen haben,
ist dadurch gekennzeichnet, daß Hydrochinon der Formel

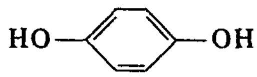

mit einer Verbindung der vorstehenden allgemeinen Formel (III) zur Reaktion gebracht wird.

Eine Verbindung der allgemeinen Formel (VII), in der M ein Alkalimetall-Atom ist, kann mittels eines üblichen Verfahrens hergestellt werden, indem das 2-(4-Hydroxyphenoxy)propionat der allgemeinen Formel (VII-i) mit einem Alkalimetall-hydroxid, -carbonat oder -alkoholat umgesetzt wird.

Die DE-OS 26 17 804 (entsprechend der JP-OS 131 545/1977) offenbart, daß 2-(4'-Phenoxyphenoxy)propionsäure-Derivate der nachstehenden Formel (A) herbizide Wirksamkeit besitzen, wobei in der Formel (A)

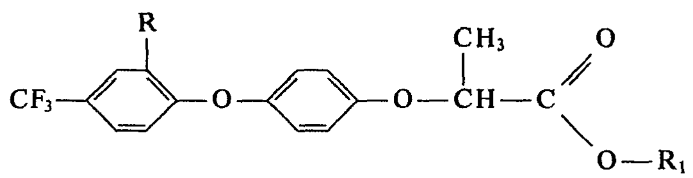

(A)

R Wasserstoff oder Halogen bezeichnet, und
$R^1$ (a) ein lineares oder verzweigtes $C_1$- bis $C_{12}$-Alkyl ist, das mono- oder polysubstituiert ist durch Cyclohexyl, Halogenophenyl, Nitrophenyl, $C_1$- bis $C_6$-Alkylphenyl, Phenoxy (das gegebenenfalls mono- bis trisubstituiert ist durch Halogen und/oder $C_1$- bis $C_4$-Alkyl, $C_5$- bis $C_6$-Alkoxy, $C_5$- bis $C_6$-Alkoxy-($C_2$- bis $C_4$)-alkoxy, $C_1$- bis $C_4$-Alkoxyethoxyethoxy, $C_1$- bis $C_4$-Acyl, eine durch die Formel

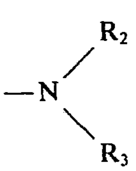

oder

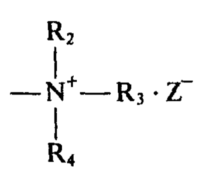

bezeichnete Gruppe oder, in 2-Stellung zu der Carboxyl-Gruppe oder einer weiter entfernten Stellung, durch Phenyl;
(b) Cyclohexenyl oder Phenyl-($C_3$- bis $C_4$)-alkenyl ist;
(c) $C_3$- bis $C_4$-Alkinyl ist (das gegebenenfalls mono- oder disubstituiert ist durch lineares oder verzweigtes $C_1$- bis $C_4$-Alkyl, Halogen, Phenyl, Halogenophenyl oder $C_1$- bis $C_4$-Alkylphenyl), vorausgesetzt, daß $R_1$ nicht unsubstituiertes Propargyl oder Butinyl bezeichnet;

(d) eine der durch die nachstehenden Formeln

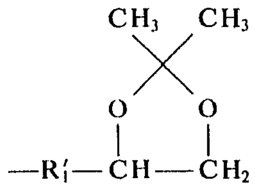

—$R_1'$—O—CO—$R_5$

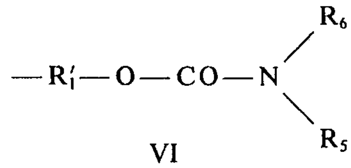

IV V VI

und —$R_1'$—$S(O)_n$—$R_6$

VII

bezeichneten Gruppen ist oder

(e) $C_1$- bis $C_2$-Alkyl ist, das durch Furyl, Tetrahydrofuryl, Pyridyl oder Oxiranyl substituiert ist;

worin

$R_2$ Wasserstoff, $C_1$- bis $C_4$-Alkyl oder $C_1$- bis $C_4$-Alkoxy bezeichnet,
$R_3$ Wasserstoff, $C_1$- bis $C_4$-Alkyl oder Phenyl bezeichnet oder
$R_2$ und $R_3$ zusammen eine 4- oder 5gliedrige gesättigte oder ungesättigte Alkylen-Kette bilden, in der eine Methylen-Gruppe gegebenenfalls ersetzt sein kann durch

—O— 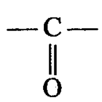 oder 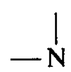—($C_1$- bis $C_4$-Alkyl),

$R_4$ Wasserstoff oder $C_1$- bis $C_2$-Alkyl bezeichnet,
Z ein anorganisches oder organisches Anion bezeichnet,
$R_1'$ ein lineares oder verzweigtes $C_1$- bis $C_{12}$-Alkylen bezeichnet,
$R_5$ Wasserstoff, $C_1$- bis $C_4$-Alkyl, $C_1$- bis $C_4$-Halogenoalkyl, Phenyl, das gegebenenfalls durch Halogen, Nitro und/oder $C_1$- bis $C_4$-Alkyl substituiert ist, oder eine Gruppe der Formel

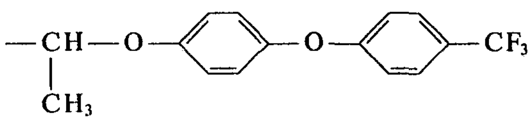 (VIII)

oder

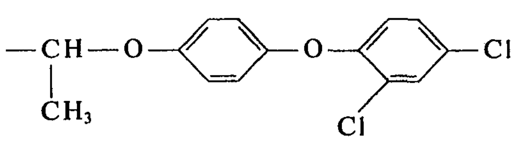 (IX)

bezeichnet,
$R_6$ $C_1$- bis $C_4$-Alkyl bezeichnet und
n für 0, 1 oder 2 steht.

$R_1$ in der Formel (A) der im Vorstehenden zitierten DE-OS (JP-OS) schließt nicht solche Verbindungen ein, die die Gruppe

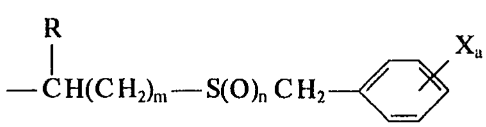

enthalten, die in der allgemeinen Formel (I), die in der vorliegenden Erfindung angegeben ist, speziell bezeichnet wird.

Die DE-OS 28 12 571 (entsprechend der JP-OS 119 476/1979) offenbart, daß Verbindungen der nachstehenden Formel (B) herbizide Wirksamkeit besitzen, wobei in der Formel (B)

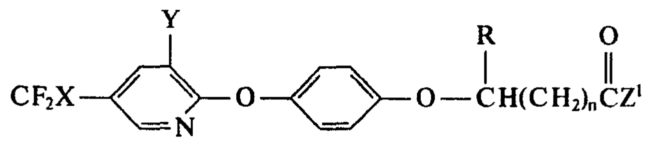

(B)

X Fluor oder Chlor bezeichnet,
Y Wasserstoff oder Chlor bezeichnet,
R Wasserstoff, Methyl oder Ethyl bezeichnet,
n für 0, 1 oder 2 steht und
$Z^1$ Hydroxy, $C_1$- bis $C_6$-Alkoxy, dessen Alkyl-Struktureinheit gegebenenfalls substituiert sein kann durch 1 bis 3 Halogene, $C_1$- bis $C_4$-Alkoxy-($C_1$- bis $C_4$)-alkoxy, $C_2$- bis $C_4$-Alkenyloxy, $C_2$- bis $C_4$-Alkinyloxy, $C_3$- bis $C_6$-Cycloalkyl, dessen Cycloalkyl-Struktureinheit gegebenenfalls substituiert sein kann durch $C_1$- bis $C_4$-Alkyl, $C_1$- bis $C_4$-Alkoxycarbonyl-($C_1$- bis $C_4$)-alkoxy, Phenoxy, dessen Phenyl-Struktureinheit gegebenenfalls substituiert sein kann durch 1 bis 3 Halogene oder $C_1$- bis $C_4$-Alkyle, Benzyloxy, Glycidyloxy, $C_1$- bis $C_4$-Alkylthio, $C_2$- bis $C_4$-Alkenylthio, Phenylthio, dessen Phenyl-Struktureinheit gegebenenfalls substituiert sein kann durch 1 bis 3 Halogene oder $C_1$- bis $C_4$-Alkyle, Amino, $C_1$- bis $C_4$-Alkylamino, $C_1$- bis $C_4$-Alkoxycarbonylmethylamino, Hydroxycarbonylmethylamino, eine Anilino-Gruppe, deren Phenyl-Struktureinheit gegebenenfalls substituiert sein kann durch 1 bis 3 Halogene, Pyridin-2-ylamino, —O— -Kation oder Halogen bezeichnet.

$Z^1$ in der Formel (B) der im Vorstehenden zitierten DE-OS (JP-OS) schließt nicht die Gruppe

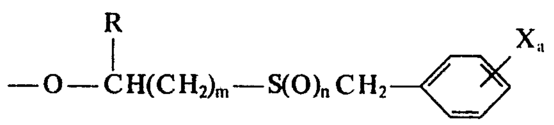

ein, die in der allgemeinen Formel (I) in der vorliegenden Erfindung speziell bezeichnet wird.

Seitens der Anmelderin wurden Forschungs- und Entwicklungsarbeiten über herbizid wirksame Verbindungen aus der Reihe der substituierten Phenoxypropionate durchgeführt. Bei diesen Untersuchungen wurde gefunden, daß die substituierten Phenoxypropionate der allgemeinen Formel (I), die in der bisher bekannten Literatur nicht beschrieben sind, sich leicht in guten Ausbeuten synthetisieren lassen und daß die Verbindungen der Formel (I) neue biologisch aktive Verbindungen sind, die eine ausgezeichnete selektive herbizide Wirkung gegen grasartige Unkräuter zeigen, ohne in nennenswertem Umfang phytotoxisch gegen auf landwirtschaftlichen Ackerflächen angebaute Nutzpflanzen zu wirken.

Wie aus der allgemeinen Formel (I) zu ersehen ist, sind die durch die allgemeine Formel (I) bezeichneten Verbindungen der vorliegenden Erfindung strukturell dadurch charakterisiert, daß in Alkylestern substituierter Phenoxypropionsäuren die Alkyl-Gruppe durch eine substituierte Benzylthio-Gruppe, eine substituierte Benzylsulfinyl-Gruppe oder eine substituierte Benzylsulfonyl-Gruppe substituiert ist.

Die seitens der Anmelderin durchgeführten Untersuchungen haben auch zu dem Befund geführt, daß die Verbindungen (I) der vorliegenden Erfindung mit einer solchermaßen speziellen Struktur hervorragende Eigenschaften besitzen, die bei analogen Verbindungen nicht zu finden sind. Beispielsweise zeigen die erfindungsgemäßen Verbindungen eine hinreichende herbizide Wirkung bei niedrigen Dosierungen und vermögen die Fortpflanzung perennierender, grasartiger Unkräuter aufgrund ihrer ausgezeichneten Nachwirkung über lange Zeiträume hinweg zu verhindern. Außerdem zeichnen sie sich durch hervorragende Selektivität aus, aufgrund derer sich eine phytotoxische Wirkung auf Nutzpflanzen vermeiden läßt.

Nach bestem Wissen der Anmelderin sind auch die 2-Halogenopropionate der im Vorstehenden angegebenen allgemeinen Formel (III) und die 2-(4-Hydroxyphenoxy)propionate und deren Alkalimetall-Salze der allgemeinen Formel (VII), die als Zwischenprodukte für die Herstellung der Verbindungen der Formel (I) mit ausgezeichneter herbizider Anwendbarkeit von Wert sind, ebenfalls neue Verbindungen, die in der bisher bekannten Literatur nicht beschrieben sind.

Ziel der vorliegenden Erfindung ist demgemäß, die substituierten Phenoxypropionate der allgemeinen Formel (I), Zwischenprodukte für diese, Verfahren zur Herstellung dieser Ester und der Zwischenprodukte sowie die Verwendung der Phenoxypropionate der Formel (I) als Herbizide verfügbar zu machen.

Diese und andere Ziele der vorliegenden Erfindung zusammen mit ihren Vorteilen sind der folgenden Beschreibung zu entnehmen.

Die erfindungsgemäßen Stoffe können mit Vorteil zur Unkrautbekämpfung eingesetzt werden, da sie eine niedrige Toxizität gegenüber warmblütigen Tieren und eine gute Selektivität für Kulturpflanzen besitzen, das heißt, bei der Anwendung in den üblichen Konzentrationen keine Phytotoxizität gegenüber Kulturpflanzen aufweisen. Insbesondere zeigen die erfindungsgemäßen Stoffe eine herausragende selektive Bekämpfungswirksamkeit, wenn sie als Mittel für die Bodenbehandlung vor dem Auflaufen und als Mittel für die Behandlung von Stengeln und Laub sowie des Bodens gegen grasartige Unkräuter verwendet werden.

Wie bereits oben dargelegt wurde, zeichnen sich die Verbindungen der Formel (I) gemäß der vorliegenden Erfindung durch hohe Sicherheit und hervorragende herbizide Wirkungen aus.

Die Verbindungen gemäß der vorliegenden Erfindung besitzen ein solches herbizides Spektrum, daß sie eine starke herbizide Wirkung gegen

Echinochloa crus-galli P. Beauv.,
Digitaria adscendens Henr.,
Eleusine indica Gaertn.,
Setaria viridis P. Beauv.,
Avena fatua,
Alopecurus aequalis var. amurensis Ohwi,
Setaria lutescens,
Agropyron repens und
Agropyron tsukushiense var. transiens Ohwi

sowie auch eine ausgezeichnete herbizide Wirkung und fortpflanzungshemmende Wirkung gegenüber anderen perennierenden Unkräutern wie

Johnson-Gras und
Cynodon dactylon Parsoon.

Die Herbizide der vorliegenden Erfindung können, ohne daß sie eine Schadwirkung ausüben, beim Anbau vieler Nutzpflanzen wie Bohnen, Baumwolle, Möhren, Kartoffeln, Rüben, Kohlgemüsen, Senf, Erdnüssen, Rettich, Tabak, Tomaten und Gurken eingesetzt werden.

Die Verbindungen der Formel (I) gemäß der vorliegenden Erfindung können beispielsweise mittels der folgenden Verfahren i), ii), iii) und iv) hergestellt werden.

Verfahren i)

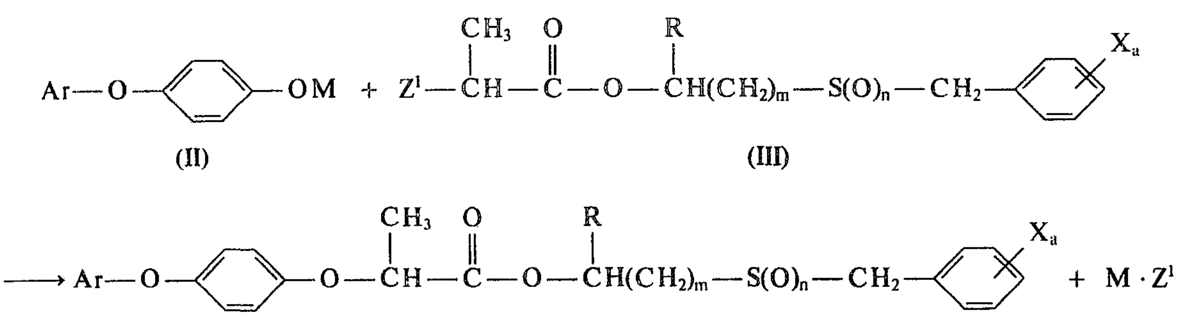

(II) (III)

(I)

(In den vorstehenden Formeln haben Ar, R, X, a, m, n, M und $Z^1$ die im Vorstehenden angegebenen Bedeutungen)

In dem obigen Reaktionsschema sind spezielle Beispiele für Y in Ar, das für die Gruppen

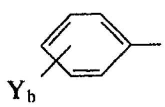

oder

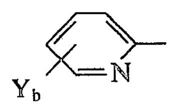

steht,

Halogen-Atome wie Fluor, Chlor, Brom und Iod sowie eine Trifluoromethyl-Gruppe, und b ist 1 oder 2.

R bezeichnet ein Wasserstoff-Atom oder eine Methyl-Gruppe.

X bezeichnet ein Wasserstoff-Atom, ein Halogen-Atom, eine Nitro-Gruppe, eine niedere Alkyl-Gruppe oder eine niedere Alkoxy-Gruppe. Die Halogen-Atome können die gleichen sein wie die oben beispielhaft genannten. Beispiele für die niedere Alkyl-Gruppe sind Methyl, Ethyl, Propyl, Isopropyl und n-(iso-, sec- oder tert-)-Butyl. Beispiele für die niedere Alkoxy-Gruppe sind niedere Alkoxy-Gruppen, deren Alkyl-Struktureinheiten gleich denjenigen sind, die im Vorstehenden beispielhaft für die niederen Alkyl-Gruppen genannt wurden.

a und m stehen jeweils für 1 oder 2, und n steht für 0, 1 oder 2.

In dem durch das vorstehende Schema dargestellten Verfahren zur Herstellung der Verbindung der allgemeinen Formel (I) zählen zu speziellen Beispielen für die Ausgangs-Verbindung der allgemeinen

Formel (II) 4-(4-Trifluoromethylphenoxy)phenol, 4-(4-Chloro-2-trifluoromethylphenoxy)phenol, 4-(5-Trifluoromethyl-2-pyridyloxy)-phenol und 4-(3,5-Dichloro-2-pyridyloxy)phenol. Ebenfalls zu nennen sind deren Salze mit Alkalimetallen wie die Li-, Na- und K-Salze.

Zu speziellen Beispielen für die Verbindung der allgemeinen Formel (III), die in gleicher Weise ein Ausgangsmaterial für die Reaktion gemäß dem vorstehenden Reaktionsschema ist, zählen die folgenden Verbindungen:

2-Benzylthioethyl-2-chloro(oder bromo)propionat,
1-Methyl-2-benzylthioethyl-2-chloro(oder bromo)propionat,
3-Benzylthiopropyl-2-chloro(oder bromo)propionat,
2-(2-Fluorbenzylthio)ethyl-2-chloro(oder bromo)-propionat,
2-(2-Chlorobenzylthio)ethyl-2-chloro(oder bromo)-propionat,
2-(4-Chlorobenzylthio)ethyl-2-chloro(oder bromo)propionat,
2-(4-Methoxybenzylthio)ethyl-2-chloro(oder bromo)propionat,
2-(3-Nitrobenzylthio)ethyl-2-chloro(oder bromo)propionat,
2-(2,4-Dichlorobenzylthio)ethyl-2-chloro(oder bromo)-propionat,
2-Benzylsulfinylethyl-2-chloro(oder bromo)propionat,
2-(4-Methylbenzylthio)ethyl-2-chloro(oder bromo)propionat und
2-Benzylsulfonylethyl-2-chloro(oder bromo)propionat.

Das vorerwähnte Herstellungsverfahren wird anhand eines typischen Beispiels im einzelnen erläutert.

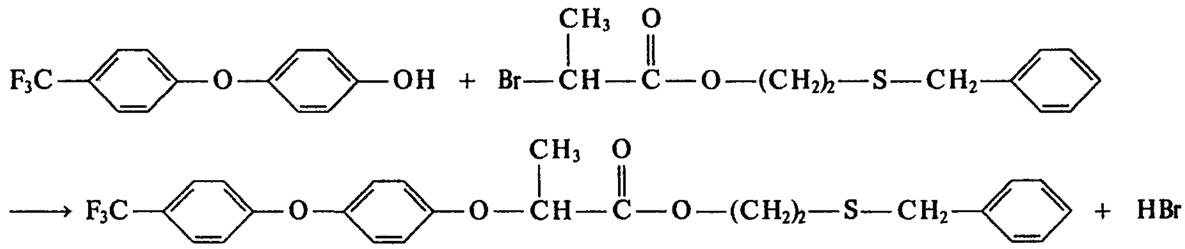

Das vorstehende Verfahren kann zweckmäßigerweise unter Verwendung eines Lösungsmittels oder Verdünnungsmittels durchgeführt werden, und zu diesem Zweck können alle inerten Lösungsmittel oder Verdünnungsmittel verwendet werden.

Beispiele für solche Lösungsmittel oder Verdünnungsmittel sind Wasser; aliphatische, alicyclische und aromatische Kohlenwasserstoffe (die gegebenenfalls chloriert sein können) wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Di-isopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Alkohole wie Methanol, Ethanol, Isopropanol, Butanol und Ethylenglycol; Ester wie Ethylacetat und Amylacetat; Säureamide wie Dimethylformamid und Dimethylacetamid; Sulfone und Sulfoxide wie Dimethylsulfoxid und Sulfolan; sowie Basen wie Pyridin.

Die Reaktion kann in Gegenwart eines säurebindenden Mittels durchgeführt werden. Beispiele für solche säurebindenden Mittel sind die Hydroxide, Carbonate, Hydrogencarbonate und Alkoholate von Alkalimetallen sowie tertiäre Amine wie Triethylamin, Diethylanilin und Pyridin, die allgemein verwendet werden.

Das Verfahren gemäß der vorliegenden Erfindung kann bei einer Temperatur innerhalb eines weiten Bereichs durchgeführt werden. Im allgemeinen wird es bei einer Temperatur zwischen −20°C und dem Siedepunkt der Mischung, vorzugsweise zwischen 0°C und 100°C durchgeführt.

Zweckmäßigerweise wird die Reaktion unter atmosphärischem Druck durchgeführt, obwohl sie auch unter erhöhtem oder vermindertem Druck durchgeführt werden kann.

Verfahren ii)

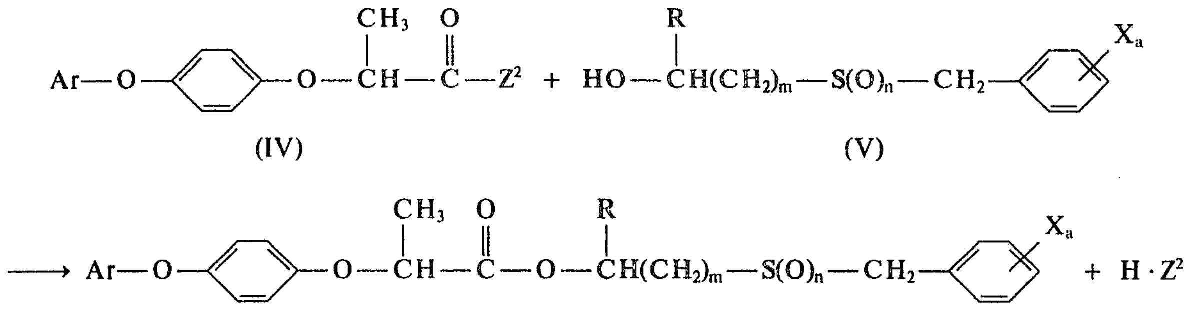

(IV) (V)

(I)

(In den obigen Formeln haben Ar, R, X, a, m, n und $Z^2$ die im Vorstehenden angegebenen Bedeutungen)

In dem durch das vorstehende Reaktionsschema dargestellten Verfahren zur Herstellung der Verbindung der allgemeinen Formel (I) sind spezielle Beispiele für die Ausgangs-Verbindung der allgemeinen Formel (IV) die folgenden:

2-[4-(4-Trifluoromethylphenoxy)phenoxy]propionylchlorid,
2-[4-(4-Chloro-2-trifluoromethylphenoxy)phenoxy]-propionylchlorid,
2-[4-(5-Trifluoromethyl-2-pyridyloxy)phenoxy]propionylchlorid und
2-[4-(3,5-Dichloro-2-pyridyloxy)phenoxy]propionylchlorid.

Die entsprechenden Bromide und freien Propionsäuren sind ebenfalls als Beispiele zu nennen.
In gleicher Weise zählen zu Beispielen für die Verbindung der Formel (V), dem anderen Ausgangsmaterial, die folgenden Verbindungen:

2-Benzylthioethanol,
1-Benzylthio-2-propanol,
3-Benzylthio-1-propanol,
2-(2-Fluorobenzylthio)ethanol,
2-(2-Chloroybenzylthio)ethanol,
2-(4-Chlorobenzylthio)ethanol,
2-(4-Methoxybenzylthio)ethanol,
2-(3-Nitrobenzylthio)ethanol,
2-(2,4-Dichlorobenzylthio)ethanol,
2-Benzylsulfinylethanol,
2-(4-Methylbenzylthio)ethanol und
2-Benzylsulfonylethanol.

Das vorerwähnte Herstellungsverfahren wird anhand eines typischen Beispiels im einzelnen erläutert.

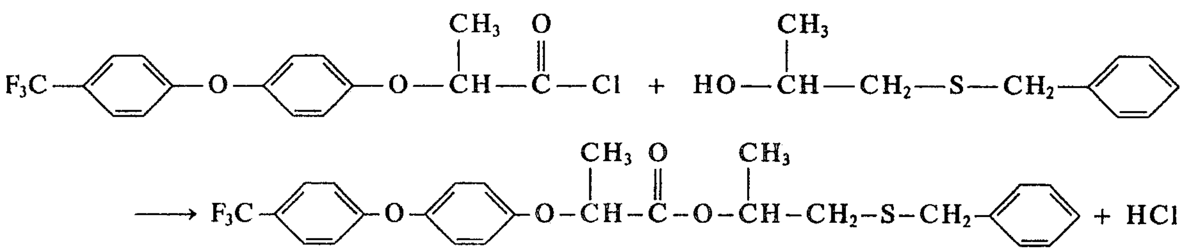

Bei Durchführung des obigen Verfahrens, vorzugsweise unter Verwendung der gleichen inerten Lösungsmittel oder Verdünnungsmittel, wie sie im Vorstehenden beispielhaft angegeben sind, jedoch mit Ausnahme von Alkoholen, kann das angestrebte Produkt in hoher Reinheit und in hoher Ausbeute erhalten werden.

Die vorstehende Reaktion kann bei einer Temperatur innerhalb eines weiten Bereichs durchgeführt werden, im allgemeinen bei einer Temperatur zwischen etwa −20°C und dem Siedepunkt der Mischung, vorzugsweise zwischen etwa 0°C und etwa 100°C. Zweckmäßigerweise wird die Reaktion unter atmosphärischem Druck durchgeführt, obwohl sie auch bei erhöhtem oder vermindertem Druck durchgeführt werden kann.

Verfahren iii)

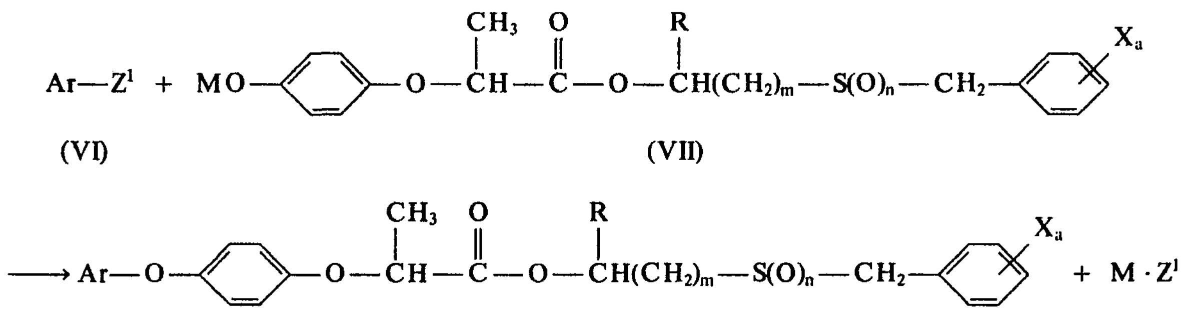

(VI) (VII)

(I)

(In den obigen Formeln haben Ar, R, X, a, m, n, M und $Z^1$ die im Vorstehenden angegebenen Bedeutungen)

Zu speziellen Beispielen für Verbindungen der allgemeinen Formel (VI), die als eines der Ausgangsmaterialien in dem Verfahren zur Herstellung der Verbindung der allgemeinen Formel (I) nach dem obigen Reaktionsschema eingesetzt werden, zählen

4-Chloro(oder Bromo)trifluoromethylbenzol,
1,4-Dichloro-2-trifluoromethylbenzol,
1-Bromo-4-chloro-3-trifluoromethylbenzol,
2-Chloro(oder Fluoro oder Bromo)-5-trifluoromethylpyridin,
2,3,5-Trichloropyridin und
2-Bromo-3,5-dichloropyridin.

In gleicher Weise zählen zu Beispielen für die Verbindung der Formel (VII), dem anderen Ausgangsmaterial, die folgenden Verbindungen:

2-Benzylthioethyl-2-(4-hydroxyphenoxy)propionat,
1-Methyl-2-benzylthioethyl-2-(4-hydroxyphenoxy)propionat,
3-Benzylthioethyl-2-(4-hydroxyphenoxy)propionat,
2-(2-Fluorobenzylthio)ethyl-2-(4-hydroxyphenoxy)propionat,
2-(2-Chlorobenzylthio)ethyl-2-(4-hydroxyphenoxy)propionat,
2-(4-Chlorobenzylthio)ethyl-2-(4-hydroxyphenoxy)propionat,
2-(4-Methoxybenzylthio)ethyl-2-(4-hydroxyphenoxy)propionat,
2-(3-Nitrobenzylthio)ethyl-2-(4-hydroxyphenoxy)propionat,
2-(2,4-Dichlorobenzylthio)ethyl-2-(4-hydroxyphenoxy)propionat,
2-Benzylsulfinylethyl-2-(4-hydroxyphenoxy)propionat,
2-(4-Methylbenzylthio)ethyl-2-(4-hydroxyphenoxy)propionat und
2-Benzylsulfonylethyl-2-(4-hydroxyphenoxy)propionat.

Die Alkalimetall-Salze wie die Li-, Na- und K-Salze dieser Verbindungen können ebenfalls als Beispiele angeführt werden.

Das vorerwähnte Herstellungsverfahren wird anhand eines typischen Beispiels im einzelnen erläutert.

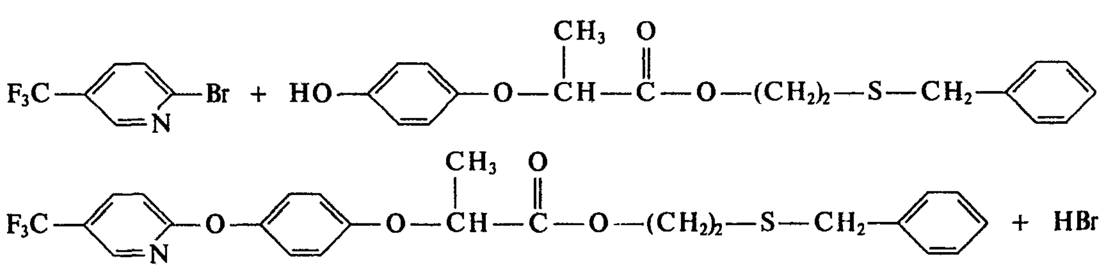

Bei Durchführung des obigen Verfahrens, vorzugsweise unter Verwendung der gleichen inerten Lösungsmittel oder Verdünnungsmittel, wie sie im Vorstehenden beispielhaft angegeben sind, kann das angestrebte Produkt in hoher Reinheit und in hoher Ausbeute erhalten werden.

Die vorstehende Reaktion kann bei einer Temperatur innerhalb eines weiten Bereichs durchgeführt werden, im allgemeinen bei einer Temperatur zwischen etwa −20°C und dem Siedepunkt der Mischung, vorzugsweise zwischen etwa 0°C und etwa 100°C. Zweckmäßigerweise wird die Reaktion

unter atmosphärischem Druck durchgeführt, obwohl sie auch bei erhöhtem oder vermindertem Druck durchgeführt werden kann.

Verfahren iv)

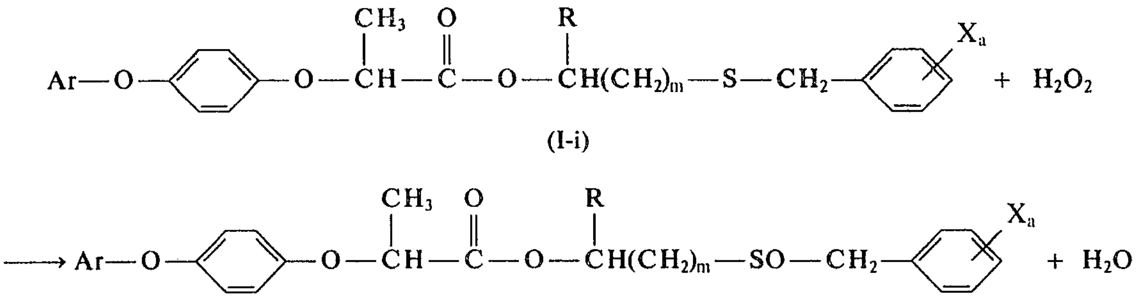

(I-i)

(I-ii)

(In den obigen Formeln haben Ar, R, X, a und m die im Vorstehenden angegebenen Bedeutungen)

In dem durch das obige Schema dargestellten Verfahren zur Herstellung der Verbindung der allgemeinen Formel (I-ii) können die Verbindungen der vorliegenden Erfindung, die mittels der im Vorstehenden beschriebenen Verfahren i), ii) und iii) synthetisiert wurden, als Beispiele für die Ausgangs-Verbindung der allgemeinen Formel (I-i) angeführt werden. Ein spezielles Beispiel ist 2-Benzylthioethyl-2-[4-(4-trifluoromethylphenoxy)-phenoxy]propionat.

Das vorerwähnte Herstellungsverfahren wird anhand eines typischen Beispiels im einzelnen erläutert.

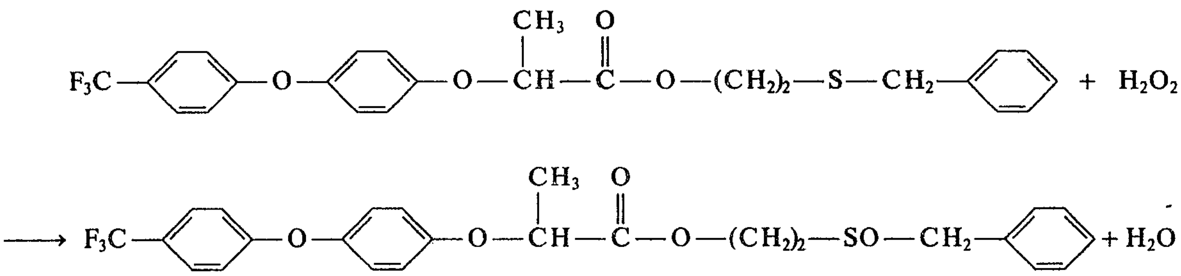

Die obige Reaktion kann vorzugsweise in Gegenwart einer organischen Säure wie Essigsäure und unter Verwendung der gleichen inerten Lösungsmittel oder Verdünnungsmittel, wie sie im Vorstehenden beispielhaft angegeben sind, durchgeführt werden, und das angestrebte Produkt kann in hoher Reinheit und in hoher Ausbeute erhalten werden.

Die vorstehende Reaktion kann bei einer Temperatur innerhalb eines weiten Bereichs durchgeführt werden, im allgemeinen bei einer Temperatur zwischen etwa −20°C und dem Siedepunkt der Mischung, vorzugsweise zwischen etwa 0°C und etwa 100°C. Zweckmäßigerweise wird die Reaktion unter atmosphärischem Druck durchgeführt, obwohl sie auch bei erhöhtem oder vermindertem Druck durchgeführt werden kann.

Die Verbindung der Formel (III), ein Zwischenprodukt für die Herstellung der Verbindung der Formel (I), kann mittels des nachstehenden Verfahrens v) hergestellt werden.

Verfahren v)

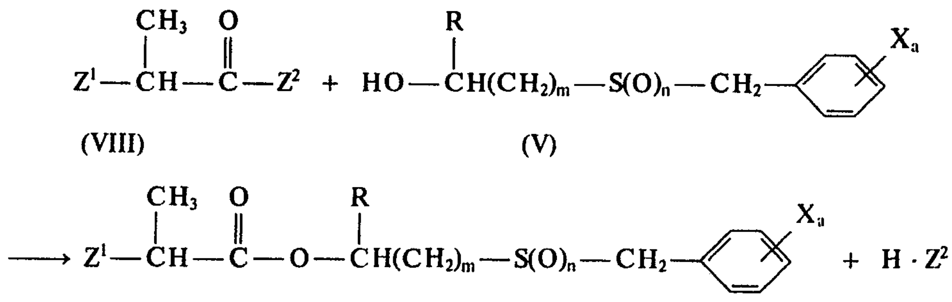

(VIII) (V)

(III)

(In den obigen Formeln haben R, X, a, m, n, $Z^1$ und $Z^2$ die im Vorstehenden angegebenen Bedeutungen)

In dem durch das obige Schema dargestellten Verfahren zur Herstellung der Verbindung der allgemeinen Formel (III) umfassen spezielle Beispiele für die Verbindung der allgemeinen Formel (VIII) 2-Chloropropionsäure und 2-Bromopropionsäure. Die entsprechenden Säurehalogenide (z. B. Chloride oder Bromide) können ebenfalls als Beispiele angeführt werden.

Spezielle Beispiele für die Verbindung der allgemeinen Formel (V) können diejenigen sein, die bereits im Vorstehenden angegeben wurden.

Das vorerwähnte Herstellungsverfahren wird anhand eines typischen Beispiels im einzelnen erläutert.

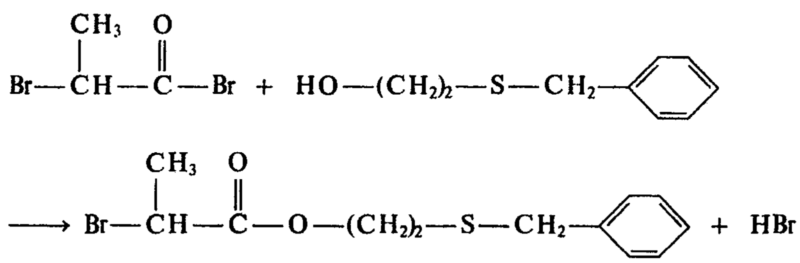

Das obige Verfahren wird vorzugsweise unter Verwendung der gleichen inerten Lösungsmittel oder Verdünnungsmittel, wie sie im Vorstehenden beispielhaft angegeben sind, jedoch mit Ausnahme der Alkohole, durchgeführt, und das angestrebte Produkt kann in hoher Reinheit erhalten werden.

Die vorstehende Reaktion kann bei einer Temperatur innerhalb eines weiten Bereichs durchgeführt werden, im allgemeinen bei einer Temperatur zwischen etwa −20°C und dem Siedepunkt der Mischung, vorzugsweise zwischen etwa 0°C und etwa 100°C. Zweckmäßigerweise wird die Reaktion unter atmosphärischem Druck durchgeführt, obwohl sie auch bei erhöhtem oder vermindertem Druck durchgeführt werden kann.

In der gleichen Weise können das 2-(4-Hydroxyphenoxy)propionat und sein Alkalimetall-Salz, die durch die Formel (VII) dargestellt werden.

Verfahren vi)

(bei dem M in der allgemeinen Formel (VII) Wasserstoff ist)

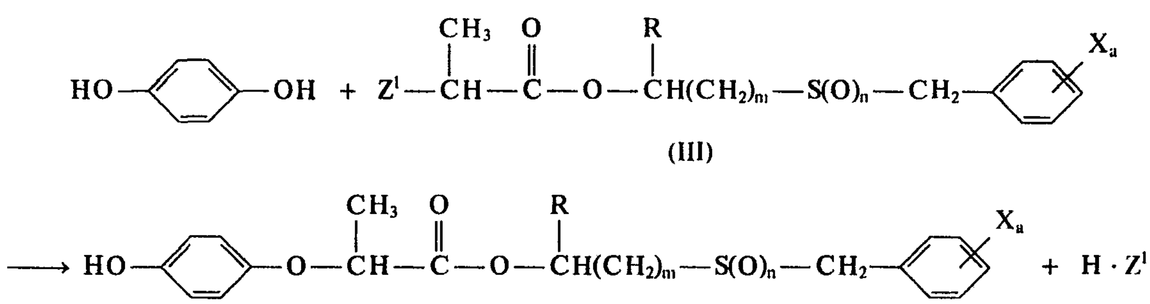

(III)

(VII-i)

(In den obigen Formeln haben R, X, a, m, n und $Z^1$ die im Vorstehenden angegebenen Bedeutungen)

In dem durch das obige Schema dargestellten Verfahren zur Herstellung der Verbindung der allgemeinen Formel (VII-i) können spezielle Beispiele für die Ausgangs-Verbindung der allgemeinen Formel (III) diejenigen sein, die bereits im Vorstehenden angegeben wurden.

Das vorerwähnte Herstellungsverfahren wird anhand eines typischen Beispiels im einzelnen erläutert.

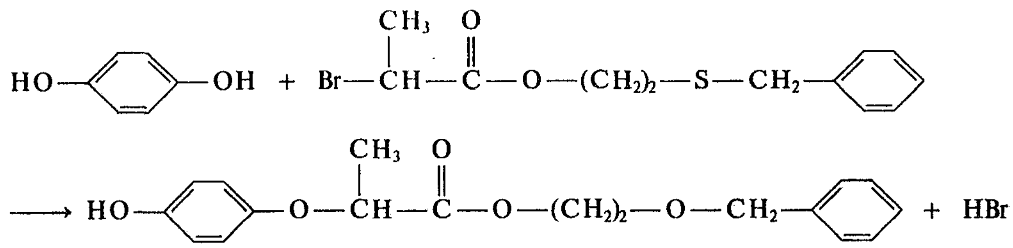

Das obige Verfahren wird vorzugsweise unter Verwendung der gleichen inerten Lösungsmittel oder Verdünnungsmittel, wie sie im Vorstehenden beispielhaft angegeben sind, durchgeführt, und das angestrebte Produkt kann in hoher Reinheit und in hoher Ausbeute erhalten werden.

Die vorstehende Reaktion kann bei einer Temperatur innerhalb eines weiten Bereichs durchgeführt werden, im allgemeinen bei einer Temperatur zwischen etwa −20°C und dem Siedepunkt der Mischung, vorzugsweise zwischen etwa 0°C und etwa 100°C. Zweckmäßigerweise wird die Reaktion unter atmosphärischem Druck durchgeführt, obwohl sie auch bei erhöhtem oder vermindertem Druck durchgeführt werden kann.

Eine Verbindung der allgemeinen Formel (VII), in der M ein Alkalimetall-Atom ist, kann hergestellt werden durch Umsetzung des erhaltenen 2-(4-Hydroxyphenoxy)propionats der allgemeinen Formel (VII-i) mit einem Alkalimetallhydroxid, -carbonat oder -alkoholat.

Spezielle Beispiele für das Alkalimetallhydroxid sind Lithiumhydroxid, Natriumhydroxid und Kaliumhydroxid. Spezielle Beispiele für das Alkalimetallcarbonat sind Natriumcarbonat und Kaliumcarbonat. Spezielle Beispiele für das Metallalkoholat sind Natrium- oder Kaliummethylat und Natrium- oder Kaliummethylat.

Für den Einsatz als Herbizid können die Verbindungen gemäß der vorliegenden Erfindung zu verschiedenen Wirkstoffpräparaten formuliert werden, wie sie unter Verwendung landwirtschaftlich unbedenklicher Hilfsstoffe durch Verfahren gewonnen werden, die bei der praktischen Herstellung von Agrochemikalien allgemein angewandt werden. Im praktischen Gebrauch werden die herbiziden Mittel in Form ihrer verschiedenen Präparate entweder unmittelbar oder nach dem Verdünnen mit Wasser auf die gewünschte Konzentration eingesetzt.

Beispiele für die landwirtschaftlich unbedenklichen Hilfsstoffe, auf die hier Bezug genommen wird, sind Verdünnungsmittel (Lösungsmittel, Streckmittel, Träger), oberflächenaktive Mittel (Lösungsvermittler, Emulgatoren, Dispergiermittel, Netzmittel), Stabilisatoren, Haftmittel, Aerosol-Treibmittel und synergistische Mittel.

Beispiele für die Lösungsmittel sind Wasser und organische Lösungsmittel, beispielsweise Kohlenwasserstoffe [z. B. n-Hexan, Petrolether, Naphtha, Erdöl-Fraktionen (z. B. Paraffinwachse, Kerosin, Leichtöle, Mittelöle, Schweröle), Benzol, Toluol und Xylole], halogenierte Kohlenwasserstoffe (z. B. Methylenchlorid, Kohlenstofftetrachlorid, Trichloroethylen, Ethylenchlorid, Ethylendichlorid, Chlorbenzol und Chloroform), Alkohole (z. B. Methylalkohol, Ethylalkohol, Propylalkohol und Ethylenglycol), Ether (z. B. Ethylester, Ethylenoxid und Dioxan), Alkohol-ether (z. B. Ethylenglycol-monomethylether), Ketone (z. B. Aceton und Isophoron), Ester (z. B. Ethylacetat und Amylacetat), Amide (z. B. Dimethylformamid und Dimethylacetamid) und Sulfoxide (z. B. Dimethylsulfoxid).

Beispiele für die Streckmittel oder Träger umfassen anorganische Pulver, beispielsweise Löschkalk, Magnesiumkalk, Gips, Calciumcarbonat, Siliciumdioxid, Perlit, Bimsstein, Calcit, Diatomeenerde, amorphes Siliciumdioxid, Aluminiumoxid, Zeolithe und Tonminerale (z. B. Pyrophyllit, Talkum, Montmorillonit, Beidellit, Vermikulit, Kaolinit und Glimmer), pflanzliche Pulver wie beispielsweise Getreidepulver, Stärkearten, verarbeitete Stärkearten, Zucker, Glucose und zerkleinerte Stengel von Pflanzen, sowie Pulver aus synthetischen Harzen wie Phenol-Harzen, Harnstoff-Harzen und Vinylchlorid-Harzen.

Beispiele für die oberflächenaktiven Mittel umfassen anionische oberflächenaktive Mittel wie Alkylschwefelsäureester (z. B. Natriumlaurylsulfat), Arylsulfonsäure-Salze (z. B. Alkylarylsulfonsäure-Salze und Natriumalkylnaphthalinsulfonate), Bernsteinsäure-Salze und Salze von Schwefelsäureestern von Polyethylenglycol-alkylarylethern, kationische oberflächenaktive Mittel wie Alkylamine (z. B. Laurylamin, Stearyltrimethylammoniumchlorid und Alkyldimethylbenzylammoniumchlorid) und Polyoxyethylenalkylamine, nicht-ionische oberflächenaktive Mittel wie Polyoxyethylenglycolether (z. B. Polyoxyethylenalkylarylether und deren Kondensationsprodukte), Polyoxyethylenglycolester (z. B. Polyoxyethylenfettsäureester) und Ester mehrwertiger Alkohole (z. B. Polyoxyethylensorbitan-monolaurat) sowie amphotere oberflächenaktive Mittel.

Beispiele für andere Hilfsstoffe umfassen Stabilisatoren, Haftmittel (z. B. landwirtschaftliche Seifen, Casein-Kalk, Natriumalginat, Polyvinylalkohol, Haftmittel vom Vinylacetat-Typ und Acryl-Haftmittel),

wirkungsverlängernde Mittel, Dispersions-Stabilisatoren (z. B. Casein, Tragant, Carboxymethylcellulose und Polyvinylalkohol) und synergistische Mittel.

Die Verbindungen der vorliegenden Erfindung können mittels der allgemein auf dem Gebiet der Agrochemikalien gebräuchlichen Verfahren zu verschiedenen Präparaten formuliert werden. Beispiele für solche Anwendungsformen sind emulgierbare Konzentrate, Öle, benetzbare Pulver, lösliche Pulver, Suspensionen, Stäubemittel, Granulate und pulvrige Mittel.

Die Herbizide gemäß der vorliegenden Erfindung können von etwa 0,001 bis etwa 100 Gew.-%, vorzugsweise etwa von 0,005 bis 95 Gew.-% der Wirkstoffe der Formel (I) enthalten.

Für den praktischen Gebrauch beträgt die geeignete Menge der aktiven Verbindung in den vorgenannten Mitteln in ihren verschiedenen Formen und gebrauchsfertigen Präparaten beispielsweise etwa 0,01 bis etwa 95 Gew.-%, vorzugsweise etwa 0,05 bis etwa 60 Gew.-%.

Der Gehalt des Wirkstoffs kann in geeigneter Weise je nach der Form des Präparats oder Mittels, dem Verfahren, Zweck, der Zeit und dem Ort seiner Anwendung, dem Zustand des Auftretens der Unkräuter etc. variiert werden.

Erforderlichenfalls können die Verbindungen gemäß der vorliegenden Erfindung weiterhin auch in Kombination mit anderen Agrochemikalien verwendet werden, beispielsweise mit Insektiziden, Fungiziden, Mitiziden, Nematiziden, Anti-Virus-Mitteln, anderen Herbiziden, Pflanzen-Wachstumsregulatoren und Lockstoffen [Organophosphorester-Verbindungen, Carbamat-Verbindungen, Dithio(oder thiol)carbamat-Verbindungen, organischen Chlor-Verbindungen, Dinitro-Verbindungen, organischen Schwefel- oder Metall-Verbindungen, Antibiotika, substituierten Diphenylether-Verbindungen, Harnstoff-Verbindungen und Triazin-Verbindungen] und/oder Düngemitteln.

Die erfindungsgemäße Wirkstoffe enthaltenden verschiedenartigen Mittel und gebrauchsfertigen Präparate können mittels verschiedener Verfahren zur Anwendung gebracht werden, wie sie allgemein für das Aufbringen von Agrochemikalien gebräuchlich sind, beispielsweise durch Dispergieren (Versprühen von Flüssigkeiten, Vernebeln, Zerstäuben, Stäuben, Streuen von Granulat, Wasser-Oberflächenbehandlung und Gießen) und Bodenbehandlung (Vermischen mit dem Boden und Streuen). Sie können auch mittels des sogenannten Ultra-Low-Volume-Sprühverfahrens aufgebracht werden. Nach diesem Verfahren kann der Wirkstoff sogar in einer Konzentration von 100% zur Anwendung gelangen.

Die Aufwandmenge pro Flächeneinheit beträgt beispielsweise etwa 0,01 bis etwa 2,0 kg/ha, vorzugsweise etwa 0,05 bis etwa 1,0 kg/ha. In besonders gelagerten Fällen können oder sollten sogar die Aufwandmengen außerhalb des angegebenen Bereichs liegen.

Gemäß der vorliegenden Erfindung kann ein herbizides Mittel zur Verfügung gestellt werden, das als Wirkstoff die Verbindung der allgemeinen Formel (I) sowie ein Verdünnungsmittel (ein Lösungsmittel und/oder ein Streckmittel und/oder einen Träger) und/oder ein oberflächenaktives Mittel und, falls weiterhin erforderlich, einen Stabilisator, ein Haftmittel, ein synergistisches Mittel etc. enthält.

Die vorliegende Erfindung betrifft weiterhin ein Verfahren zur Unkrautbekämpfung, das darin besteht, daß eine Verbindung der allgemeinen Formel (I) allein oder in Form einer Mischung mit einem Verdünnungsmittel (einem Lösungsmittel und/oder einem Streckmittel und/oder einem Träger) und/oder einem oberflächenaktiven Mittel und, falls weiterhin erforderlich, einem Stabilisator, einem Haftmittel, einem synergistischen Mittel etc. auf Unkräuter und/oder ihren Lebensraum aufgebracht wird.

Die vorliegende Erfindung wird durch die folgenden Beispiele näher erläutert, ist jedoch nicht auf diese beschränkt.

### Beispiel 1

### Benetzbares Pulver

15 Teile der erfindungsgemäßen Verbindung Nr. 1, 80 Teile eines Gemisches (1 : 5) aus Weißruß (feinpulvrigem wasserhaltigen amorphen Siliciumoxid) und Tonpulver, 2 Teile Natriumalkylbenzolsulfonat und 3 Teile Natriumalkylnaphthalinsulfonat/Formaldehyd-Kondensat werden pulverisiert und zu einem benetzbaren Pulver vermischt. Dieses wird mit Wasser verdünnt und auf Unkräuter und/oder ihren Lebensraum aufgebracht.

### Beispiel 2

### Emulgierbares Konzentrat

30 Teile der erfindungsgemäßen Verbindung Nr. 2, 55 Teile Xylol, 8 Teile Polyoxyethylen-alkylphenylether und 7 Teile Calciumalkylbenzolsulfonat werden unter Rühren miteinander vermischt, wodurch ein emulgierbares Konzentrat hergestellt wird. Dieses wird mit Wasser verdünnt und auf Unkräuter und/oder ihren Lebensraum aufgebracht.

Beispiel 3

Stäubemittel

2 Teile der erfindungsgemäßen Verbindung Nr. 3 und 98 Teile Tonpulver werden pulverisiert und gemischt, wodurch ein Stäubemittel hergestellt wird. Dieses wird auf Unkräuter und/oder ihren Lebensraum aufgebracht.

Beispiel 4

Stäubemittel

Die erfindungsgemäße Verbindung Nr. 4 (1,5 Teile), 0,5 Teile Isopropylhydrogenphosphat und 98 Teile Tonpulver wurden pulverisiert und gemischt, wodurch ein Stäubemittel hergestellt wurde. Dieses wird auf Unkräuter und/oder ihren Lebensraum aufgebracht.

Beispiel 5

Granulat

Wasser (25 Teile) wird zu einer Mischung aus 10 Teilen erfindungsgemäßen Verbindung Nr. 5, 30 Teilen Bentonit (Montmorillonit), 58 Teilen Talkum und 2 Teilen Ligninsulfonat zugesetzt, und das Gemisch wird gut geknetet. Die Mischung wird mittels eines Extruder-Granulators zu einem Granulat mit einer Korngröße von 0,43 bis 2,0 mm (10 bis 40 mesh) verarbeitet und bei 40°C bis 50°C getrocknet, wodurch ein Granulat gebildet wird. Dieses wird auf Unkräuter und/oder ihren Lebensraum aufgebracht.

Beispiel 6

Granulat

Ein Drehmischer wird mit 95 Teilen Tonmineral-Teilchen mit einer Teilchengrößen-Verteilung im Bereich von 0,2 bis 2 mm beschickt, und unter Drehen des Mischers werden 5 Gew.-Teile der öligen erfindungsgemäßen Verbindung Nr. 6 zur gleichmäßigen Benetzung auf die Tonmineral-Teilchen aufgesprüht. Das erhaltene Granulat wird auf Unkräuter und/oder Ihren Lebensraum aufgebracht.

Die ausgezeichnete Wirkung der Verbindungen gemäß der vorliegenden Erfindung ist aus den Ergebnissen der im Folgenden beschriebenen Tests zu entnehmen.

Test-Beispiel 1

Test der Vorauflauf-Bodenbehandlung an Acker-Unkräutern und -Nutzpflanzen

(1) Herstellung der Zusammensetzungen der aktiven Verbindungen

Träger: 5 Gew.-Teile Aceton;
Emulgator: 1 Gew.-Teil Benzyloxypolyglycolether.

Jede der Zusammensetzungen wurde durch Vermischen von 1 Gew.-Teil jeweils einer der in der Tabelle 1 aufgeführten wirksamen Verbindungen mit dem Träger und dem Emulgator hergestellt, wodurch ein emulgierbares Konzentrat erhalten wurde. Eine vorher festgelegte Menge des emulgierbaren Konzentrats wurde mit Wasser verdünnt, wodurch ein Test-Präparat erhalten wurde.

(2) Test-Verfahren

In einem Gewächshaus wurden Samen von Erdnüssen, Garten-Erbsen, Sojabohnen und Baumwolle in Töpfe (1000 $cm^2$) eingesät, die mit Ackerboden gefüllt waren. Boden, der Samen von Agropyron repens, Echinochloa crus-galli P. Beauv. und Setaria lutescens enthielt, wurde in einer Tiefe von 1 cm darüber gegeben.

Einen Tag nach der Einsaat und dem Bedecken mit dem Boden wurden 10 ml jedes Test-Präparats mit einer Konzentration desselben von 200 ppm gleichmäßig auf die Oberflächenschicht des Bodens in jedem der Test-Töpfe aufgesprüht.

Vier Wochen nach Aufsprühen des Präparats wurden die herbizide Wirkung und die Phytotoxizität der Test-Präparate untersucht.

Bewertung der herbiziden Wirkung (Unkrautvernichtungsrate bezogen auf die unbehandelten Flächen):

| | | |
|---|---|---|
| 10 | | 100% (vollständig verdorrt) |
| 9 | mindestens | 90%, jedoch weniger als 100% |
| 8 | mindestens | 80%, jedoch weniger als 90% |
| 7 | mindestens | 70%, jedoch weniger als 80% |
| 6 | mindestens | 60%, jedoch weniger als 70% |
| 5 | mindestens | 50%, jedoch weniger als 60% |
| 4 | mindestens | 40%, jedoch weniger als 50% |
| 3 | mindestens | 30%, jedoch weniger als 40% |
| 2 | mindestens | 20%, jedoch weniger als 30% |
| 1 | mindestens | 10%, jedoch weniger als 20% |
| 0 | weniger als | 10% (keine herbizide Wirkung) |

Bewertung der Phytotoxizität (Phytotoxizitätsrate bezogen auf die unbehandelten Flächen):

| | | |
|---|---|---|
| 10 | mindestens 90% | (Ausrottung) |
| 9 | mindestens 80%, | jedoch weniger als 90% |
| 8 | mindestens 70%, | jedoch weniger als 80% |
| 7 | mindestens 60%, | jedoch weniger als 70% |
| 6 | mindestens 50%, | jedoch weniger als 60% |
| 5 | mindestens 40%, | jedoch weniger als 50% |
| 4 | mindestens 30%, | jedoch weniger als 40% |
| 3 | mindestens 20%, | jedoch weniger als 30% |
| 2 | mindestens 10%, | jedoch weniger als 20% |
| 1 | mehr als 0%, | jedoch weniger als 10% |
| 0 | 0% | (keine Phytotoxizität) |

Die Testergebnisse sind in der Tabelle 1 aufgeführt.

Tabelle 1

| Verbindung Nr. | Menge des Wirkstoffs (kg/ha) | Herbizide Wirkung Unkraut Agropyron repens | Echinochloa crusgalli | Setaria lutescens | Phytotoxizität Nutzpflanzen Erdnuß | Gartenerbse | Baumwolle | Sojabohne |
|---|---|---|---|---|---|---|---|---|
| 1 | 0,2 | 10 | 10 | 10 | 0 | 0 | 0 | 0 |
| 3 | 0,2 | 10 | 10 | 10 | 0 | 0 | 0 | 0 |
| 4 | 0,2 | 10 | 10 | 10 | 0 | 0 | 0 | 0 |
| 12 | 0,2 | 10 | 10 | 10 | 0 | 0 | 0 | 0 |
| 20 | 0,2 | 10 | 10 | 10 | 0 | 0 | 0 | 0 |
| 22 | 0,2 | 10 | 10 | 10 | 0 | 0 | 0 | 0 |
| Vergleich: | | | | | | | | |
| A-1 | 0,2 | 3 | 4 | 4 | 0 | 0 | 0 | 0 |

Anmerkungen zu Tabelle 1:

1. Die Nummern der Verbindungen entsprechen denjenigen, die in den Synthese-Beispielen und in den nachstehenden Tabellen 7, 8 und 9 angegeben sind.
2. Die zum Vergleich herangezogene Verbindung A-1 ist

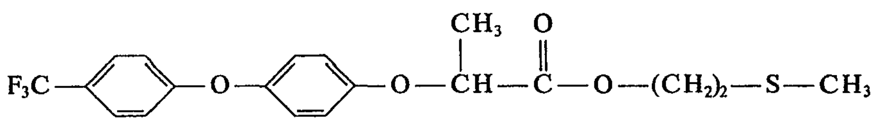

(die Verbindung, die in der DE-OS 2 617 804 beschrieben ist).

Test-Beispiel 2

Test der Stengel- und Laub-Behandlung an Acker-Unkräutern und -Nutzpflanzen

In einem Gewächshaus wurden Samen von Sojabohnen, Rettichen und Rüben in Töpfe (2000 $cm^2$) eingesät, die mit Ackerboden gefüllt waren. Boden der Samen von Panicum crus-galli, Digitaria adscendens Henr., Eleusine Indica Gaertn., Setaria viridis P. Beauv., Avena fatua und Alopecurus aequalis var. amurensis Ohwi enthielt, wurde in einer Tiefe von 1 cm darüber gegeben.

Zehn Tage nach der Einsaat und dem Bedecken mit dem Boden (als die Unkräuter sich im Durchschnitt in ihrem Zweiblatt-Stadium und Rettich-, Sojabohnen- und Rübenpflanzen sich im frühen Stadium der Entwicklung ihrer Hauptblätter befanden) wurden 20 ml jedes Test-Präparats mit einer Konzentration desselben von 100 ppm, das in gleicher Weise wie in Test-Beispiel 1 hergestellt worden war, gleichmäßig auf die Stengel und Blätter der Pflanzen gesprüht.

Drei Wochen nach dem Sprühen wurden die herbizide Wirkung und die Phytotoxizität der Test-Verbindungen in der gleichen Weise wie in dem Test-Beispiel 1 untersucht.

Die Ergebnisse sind in der Tabelle 2 aufgeführt.

Tabelle 2

| Verbindung Nr. | Menge des Wirkstoffs (kg/ha) | Herbizide Wirkung Unkräuter | | | | | | Phytotoxizität Nutzpflanzen | | |
|---|---|---|---|---|---|---|---|---|---|---|
| | | Panicum crus-galli | Digitaria adscendens | Eleusine indica | Setaria viridis | Avena fatua | Alopecurus aequalis | Sojabohne | Rettich | Rübe |
| 2 | 0,1 | 10 | 10 | 10 | 10 | 10 | 10 | 0 | 0 | 0 |
| 5 | 0,1 | 10 | 10 | 10 | 10 | 10 | 10 | 0 | 0 | 0 |
| 6 | 0,1 | 10 | 10 | 10 | 10 | 10 | 10 | 0 | 0 | 0 |
| 7 | 0,1 | 10 | 10 | 10 | 10 | 10 | 10 | 0 | 0 | 0 |
| 8 | 0,1 | 10 | 10 | 10 | 10 | 10 | 10 | 0 | 0 | 0 |
| 9 | 0,1 | 10 | 10 | 10 | 10 | 10 | 10 | 0 | 0 | 0 |
| 11 | 0,1 | 10 | 10 | 10 | 10 | 10 | 10 | 0 | 0 | 0 |
| 12 | 0,1 | 10 | 10 | 10 | 10 | 10 | 10 | 0 | 0 | 0 |
| 13 | 0,1 | 10 | 10 | 10 | 10 | 10 | 10 | 0 | 0 | 0 |
| 14 | 0,1 | 10 | 10 | 10 | 10 | 10 | 10 | 0 | 0 | 0 |
| 16 | 0,1 | 10 | 10 | 10 | 10 | 10 | 10 | 0 | 0 | 0 |
| 18 | 0,1 | 10 | 10 | 10 | 10 | 10 | 10 | 0 | 0 | 0 |
| 19 | 0,1 | 10 | 10 | 10 | 10 | 10 | 10 | 0 | 0 | 0 |
| 20 | 0,1 | 10 | 10 | 10 | 10 | 10 | 10 | 0 | 0 | 0 |
| 21 | 0,1 | 10 | 10 | 10 | 10 | 10 | 10 | 0 | 0 | 0 |
| 22 | 0,1 | 10 | 10 | 10 | 10 | 10 | 10 | 0 | 0 | 0 |
| 23 | 0,1 | 10 | 10 | 10 | 10 | 10 | 10 | 0 | 0 | 0 |
| 24 | 0,1 | 10 | 10 | 10 | 10 | 10 | 10 | 0 | 0 | 0 |
| Vergleich: | | | | | | | | | | |
| A-1 | 0,1 | 3 | 2 | 2 | 3 | 1 | 3 | 0 | 0 | 0 |
| A-2 | 0,1 | 3 | 1 | 1 | 2 | 1 | 2 | 0 | 0 | 0 |

Anmerkungen zu Tabelle 2:

1. Die Nummern der Verbindungen entsprechen denjenigen, die in den Synthese-Beispielen und in den nachstehenden Tabellen 7, 8 und 9 angegeben sind.
2. Die zum Vergleich herangezogene Verbindung A-1 ist die gleiche wie in Tabelle 1 (vgl. Anmerkung).
3. Die zum Vergleich herangezogene Verbindung A-2 ist

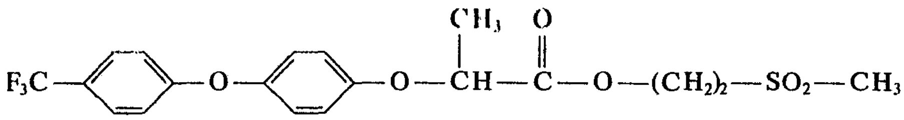

(die Verbindung, die in der DE-OS 2 617 804 beschrieben ist).

Test-Beispiel 3

Test der herbiziden Wirkung und der Hemmwirkung auf die Fortpflanzung gegen Agropyron tsukushiense var. transiens Ohwi

Ein Weg zwischen Reisfeldern, auf dem Agropyron tsukushiense in Büscheln wuchs, wurde in Teilflächen von 1 $m^2$ unterteilt. Auf jeder Test-Fläche wurden 100 ml jedes Test-Präparats mit einer Konzentration von 200 ppm auf die Stengel und Blätter von Agropyron tsukushiense aufgesprüht. Zwanzig Tage nach dem Sprühen wurde die herbizide Wirkung des Test-Präparates in der gleichen Weise wie in Test-Beispiel 1 geprüft. Weiterhin wurde 40 und 60 Tage nach dem Sprühen die Hemmwirkung auf die Fortpflanzung von Agropyron tsukushiense untersucht.

Bewertung der Hemmwirkung auf die Fortpflanzung (Rate der Hemmwirkung auf die Fortpflanzung, bezogen auf die unbehandelten Flächen):

| | | |
|---|---|---|
| 10 | | 100% (vollständige Hemmung) |
| 9 | mindestens | 90%, jedoch weniger als 100% |
| 8 | mindestens | 80%, jedoch weniger als 90% |
| 7 | mindestens | 70%, jedoch weniger als 80% |
| 6 | mindestens | 60%, jedoch weniger als 70% |
| 5 | mindestens | 50%, jedoch weniger als 60% |
| 4 | mindestens | 40%, jedoch weniger als 50% |
| 3 | mindestens | 30%, jedoch weniger als 40% |
| 2 | mindestens | 20%, jedoch weniger als 30% |
| 1 | mindestens | 10%, jedoch weniger als 20% |
| 0 | weniger als | 10% (keine Hemmwirkung) |

Die Ergebnisse sind in der Tabelle 3 aufgeführt.

Tabelle 3

| Verbindung Nr. | Menge des Wirkstoffs (kg/ha) | Herbizide Wirkung 20 Tage nach dem Sprühen | Hemmwirkung auf die Fortpflanzung 40 Tage | 60 Tage |
|---|---|---|---|---|
| 1 | 0,2 | 10 | 10 | 10 |
| 2 | 0,2 | 10 | 10 | 10 |
| 12 | 0,2 | 10 | 10 | 10 |
| 23 | 0,2 | 10 | 10 | 10 |
| Vergleich: | | | | |
| A-3 | 0,2 | 4 | 2 | 0 |
| B-1 | 0,2 | 6 | 2 | 0 |

Anmerkung zu Tabelle 3:

1. Die Nummern der Verbindungen entsprechen denjenigen, die in den Synthese-Beispielen und in den nachstehenden Tabellen 7 und 9 angegeben sind.
2. Die zum Vergleich herangezogene Verbindung A-3 ist

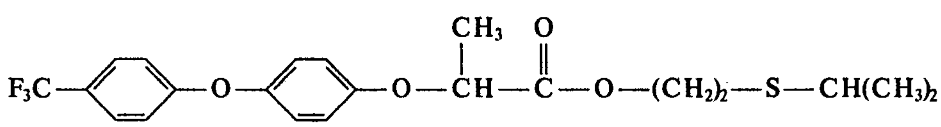

(die Verbindung, die in der DE-OS 2 617 804 beschrieben ist).

3. Die zum Vergleich herangezogene Verbindung B-1 ist

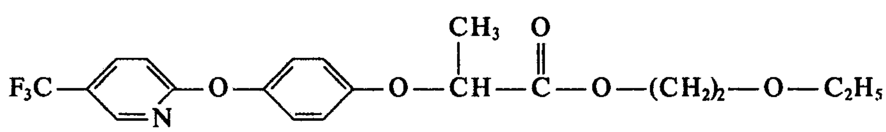

(die Verbindung, die in der DE-OS 2 812 571 beschrieben ist).

Test-Beispiel 4

Test der herbiziden Wirkung und der Hemmwirkung auf die Fortpflanzung gegen Cynodon dactylon Persoon

Eine landwirtschaftlich genutzte Ackerfläche, auf der Cynodon dactylon in Büscheln wuchs, wurde in Teilflächen von 1 $m^2$ unterteilt. Das Unkraut wurde mit jedem Test-Präparat in der gleichen Dosierung wie in Test-Beispiel 3 behandelt.

Zwanzig Tage nach dem Sprühen wurde die herbizide Wirkung geprüft. Weiterhin wurde 40 und 60 Tage nach dem Sprühen die Hemmwirkung untersucht. Die Auswertung der Ergebnisse dieses Tests erfolgte in der gleichen Weise wie im Vorstehenden beschrieben.

Die Ergebnisse sind in der Tabelle 4 aufgeführt.

Tabelle 4

| Verbindung Nr. | Menge des Wirkstoffs (kg/ha) | Herbizide Wirkung 20 Tage nach dem Sprühen | Hemmwirkung auf die Fortpflanzung 40 Tage | 60 Tage |
|---|---|---|---|---|
| 3 | 0,2 | 10 | 10 | 10 |
| 21 | 0,2 | 10 | 10 | 10 |
| 22 | 0,2 | 10 | 10 | 10 |
| Vergleich: | | | | |
| A-3 | 0,2 | 3 | 1 | 0 |
| B-1 | 0,2 | 6 | 3 | 0 |

Anmerkung zu Tabelle 4:

1. Die Nummern der Verbindungen entsprechen denjenigen, die in den Synthese-Beispielen und in den nachstehenden Tabellen 7 und 9 angegeben sind.
2. Die zum Vergleich herangezogenen Verbindungen A-3 und B-1 entsprechen den Angaben in der Anmerkung zu Tabelle 3.

Die folgenden Synthese-Beispiele erläutern speziell das Verfahren zur Herstellung des 2-Halogenopropionats der allgemeinen Formel (III).

Synthese-Beispiel 1

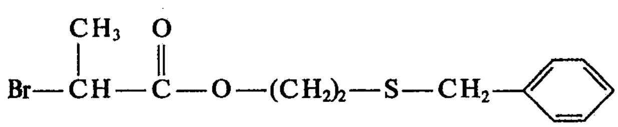

(Verbindung Nr. III-1)

Eine Lösung von 21,6 g 2-Bromopropionylbromid in 30 ml Toluol wurde tropfenweise zu einer Lösung von 10,1 g Triethylamin und 16,8 g 2-Benzylthioethanol in 150 ml Toluol hinzugefügt. Nach der Zugabe wurde die Mischung weiter 2 h bei Raumtemperatur gerührt. Die Mischung wurde nacheinander mit einer 1-proz. wäßrigen Natriumhydroxid-Lösung und mit Wasser gewaschen. Nach dem Trocknen wurde das Toluol unter vermindertem Druck abdestilliert, wonach 28,8 g 2-Benzylthioethyl-2-bromopropionat als farbloses Produkt erhalten wurden; $n_D^{20} = 1,5570$.

Nach dem im Synthese-Beispiel 1 angegebenen Verfahren wurden auch die in der Tabelle 5 aufgeführten Verbindungen der allgemeinen Formel (III) hergestellt.

Tabelle 5

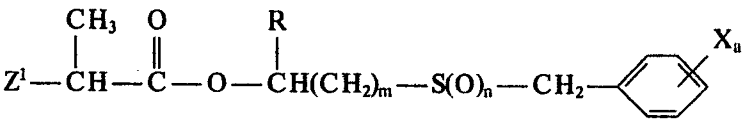

| Verbindung Nr. | $Z^1$ | R | m | n | $X_a$ | Physikal. Konstante |
|---|---|---|---|---|---|---|
| III-2 | Br | H | 1 | 0 | 2-F | $n_D^{20}$ 1.5422 |
| III-3 | Br | H | 1 | 0 | 4-Cl | $n_D^{20}$ 1.5650 |
| III-4 | Br | H | 1 | 0 | 2,4-$Cl_2$ | $n_D^{20}$ 1.5729 |
| III-5 | Br | H | 1 | 0 | 4-$OCH_3$ | $n_D^{20}$ 1.5600 |
| III-6 | Br | H | 1 | 0 | 3-$NO_2$ | $n_D^{20}$ 1.5749 |
| III-7 | Br | H | 1 | 0 | 4-$CH_3$ | $n_D^{20}$ 1.5538 |
| III-8 | Br | H | 1 | 1 | H | Öl |
| III-9 | Br | $-CH_3$ | 1 | 0 | H | $n_D^{20}$ 1.5465 |
| III-10 | Br | H | 2 | 0 | H | $n_D^{20}$ 1.5510 |
| III-11 | Cl | H | 1 | 0 | H | $n_D^{20}$ 1.5451 |
| III-12 | Cl | H | 1 | 2 | H | Öl |
| III-13 | Cl | $-CH_3$ | 1 | 0 | H | $n_D^{20}$ 1.5387 |
| III-14 | Cl | H | 1 | 0 | 2-F | $n_D^{20}$ 1.5330 |
| III-15 | Cl | H | 1 | 0 | 2-Cl | Öl |

Die folgenden Synthese-Beispiele erläutern speziell das Verfahren zur Herstellung des 2-(4-Hydroxyphenoxy)propionats der allgemeinen Formel (VII-i).

## Synthese-Beispiel 2

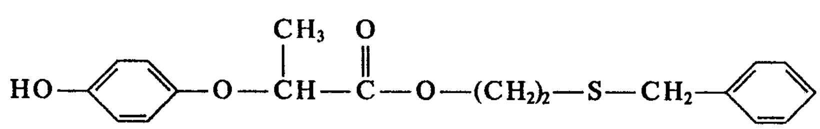

(Verbindung Nr. VII-i-1)

Hydrochinon (12,1 g) wurde in 60 ml trockenem Dimethylformamid gelöst, und unter Einblasen von Stickstoff-Gas in die Lösung wurden 31,7 g Kaliumcarbonat hinzugefügt.

Unter Rühren wurde die Mischung 1 h auf 90°C bis 95°C erhitzt. Die Temperatur im Inneren des Reaktors wurde auf 60°C erniedrigt, und 30,3 g 2-Benzylthioethyl-2-bromopropionat wurden bei dieser Temperatur tropfenweise hinzugegeben. Nach der Zugabe wurde die Mischung weiter 2 h auf 90°C erhitzt. Die Reaktionsmischung wurde auf Raumtemperatur abgekühlt und dann in Eiswasser gegossen. Der pH der Mischung wurde auf 7 eingestellt, und sie wurde mit 100 ml Chloroform extrahiert. Das Chloroform wurde abdestilliert, wonach 25,2 g 2-Benzylethyl-2-(4-hydroxyphenoxy)propionat als braunes Öl erhalten wurden; $n_D^{20}$ = 1,5706.

Nach analogen Verfahren wie in dem Synthese-Beispiel 2 wurden die in der Tabelle 6 aufgeführten Verbindungen der allgemeinen Formel (VII-i) hergestellt.

Tabelle 6

| Verbindung Nr. | Ausgangs-material | Ausgangsmaterial | Produkt der allgemeinen Formel (VII-i) |
|---|---|---|---|
| VII-i-2 | Hydrochinon | 2-(2-Fluorobenzylthio)ethyl-2-bromopropionat | 2-(2-Fluorobenzylthio)ethyl-2-(4-hydroxyphenoxy)propionat $n_D^{20} = 1,5585$ |
| VII-i-3 | Hydrochinon | 2-(4-Chlorobenzylthio)ethyl-2-bromopropionat | 2-(4-Chlorobenzylthio)ethyl-2-(4-hydroxyphenoxy)propionat $n_D^{20} = 1,5789$ |
| VII-i-4 | Hydrochinon | 2-(2,4-dichlorobenzyl)thio-ethyl-2-bromopropionat | 2-(2,4-Dichlorobenzylthio)-ethyl-2-(4-hydroxyphenoxy)-propionat |
| VII-i-5 | Hydrochinon | 2-(4-Methoxybenzylthio)-ethyl-2-bromopropionat | 2-(4-Methoxybenzylthio)-ethyl-2-(4-hydroxyphenoxy)-propionat |
| VII-i-6 | Hydrochinon | 2-(3-Nitrobenzylthio)ethyl-2-bromopropionat | 2-(3-nitrobenzylthio)ethyl-2-(4-hydroxyphenoxy)propionat |
| VII-i-7 | Hydrochinon | 2-(4-Methylbenzylthio)ethyl-2-bromopropionat | 2-(4-Methylbenzylthio)ethyl-2-(4-hydroxyphenoxy)propionat |
| VII-i-8 | Hydrochinon | 2-Benzylsulfinylethyl-2-bromopropionat | 2-Benzylsulfinylethyl-2-(4-hydroxyphenoxy)propionat |
| VII-i-9 | Hydrochinon | 1-Methyl-2-benzylthioethyl-2-bromopropionat | 1-Methyl-2-benzylthioethyl-2-(4-hydroxyphenoxy)propionat $n_D^{20} = 1,5687$ |
| VII-i-10 | Hydrochinon | 3-Benzylthiopropyl-2-bromopropionat | 3-Benzylthiopropyl-2-(4-hydroxyphenoxy)propionat $n_D^{19} = 1,5672$ |
| VII-i-11 | Hydrochinon | 2-(2-Chlorobenzylthio)ethyl-2-chloropropionat | 2-(2-Chlorobenzylthio)ethyl-2-(4-hydroxyphenoxy)propionat |
| VII-i-12 | Hydrochinon | 2-Benzylsulfonylethyl-2-chloropropionat | 2-Benzylsulfonylethyl-2-(4-hydroxyphenoxy)propionat |

Die folgenden Synthese-Beispiele erläutern speziell die Verfahren zur Herstellung des substituierten Phenoxypropionats der allgemeinen Formel (I).

Synthese-Beispiel 3

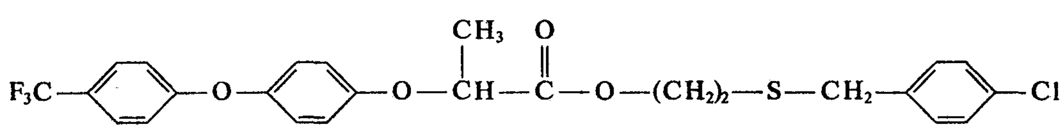

(Verbindung Nr. 1)

Eine Mischung aus 25,4 g 4-(4-Trifluoromethylphenoxy)-phenol, 14,5 g wasserfreiem Kaliumcarbonat, 33,8 g 2-(4-Chlorobenzylthio)ethyl-2-bromopropionat und 150 ml trockenem Acetonitril wurde unter kräftigem Rühren 3 h zum Rückfluß erhitzt. Nach der Reaktion wurde das Acetonitril unter vermindertem Druck abdestilliert. Toluol (150 ml) wurde zu dem Rückstand hinzugefügt. Die Toluol-Schicht wurde nacheinander mit einer 1proz. wäßrigen Natriumhydroxid-Lösung und mit Wasser gewaschen. Das Toluol wurde unter vermindertem Druck abdestilliert, wonach 46,4 g 2-(4-Chloro-

benzylthio)ethyl-2-[4-(4-trifluoromethylphenoxy)phenoxy]propionat als farbloses Öl erhalten wurden; $n_D^{20}$ = 1,5555.

Nach analogen Verfahren wie in dem Synthese-Beispiel 3 wurden die in der Tabelle 7 aufgeführten Verbindungen der allgemeinen Formel (I) hergestellt.

Tabelle 7

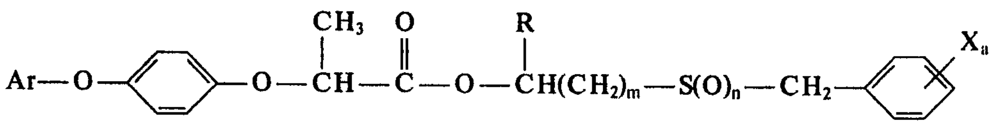

| Verbindung Nr. | Ar | R | m | n | $X_n$ | Physikalische Konstante |
|---|---|---|---|---|---|---|
| 2 | 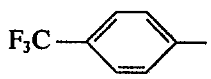 | H | 1 | 0 | H | $n_D^{20}$ 1.5495 |
| 3 | 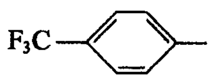 | $-CH_3$ | 1 | 0 | H | $n_D^{20}$ 1.5436 |
| 4 | 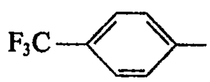 | H | 2 | 0 | H | $n_D^{20}$ 1.5470 |
| 5 | 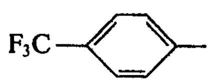 | H | 1 | 0 | 2-F | $n_D^{20}$ 1.5425 |
| 6 | 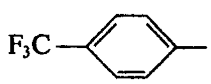 | H | 1 | 0 | 2-Cl | $n_D^{20}$ 1.5523 |
| 7 | 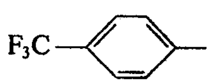 | H | 1 | 0 | 4-$OCH_3$ | $n_D^{20}$ 1.5520 |
| 8 | 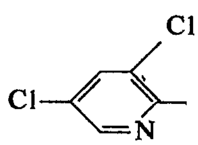 | H | 1 | 0 | 4-$OCH_3$ | $n_D^{20}$ 1.5927 |
| 9 | 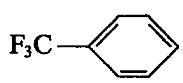 | H | 1 | 0 | 2,4-$Cl_2$ | $n_D^{20}$ 1.5600 |
| 10 | 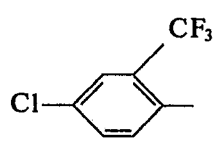 | H | 1 | 0 | H | $n_D^{20}$ 1.5583 |
| 11 | 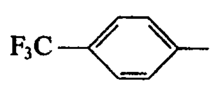 | H | 1 | 2 | H | Schmp. 126–127°C |

Synthese-Beispiel 4

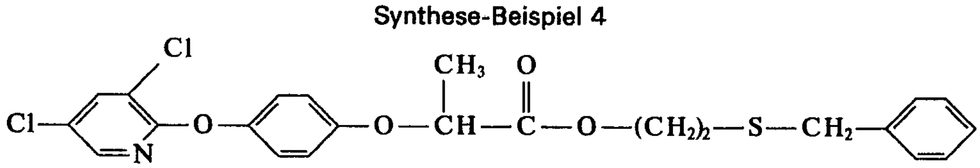

(Verbindung Nr. 12)

Eine Lösung von 34,7 g 2-[4-(3,5-Dichloro-2-pyridyloxy)phenoxy]propionylchlorid in 80 ml Toluol wurde tropfenweise bei 0°C bis 10°C zu einer Lösung von 16,8 g 2-Benzylthioethanol und 10,1 g Triethylamin in 100 ml Toluol hinzugefügt. Nach der Zugabe wurde die Mischung weiter 1 h bei 30°C bis

40°C gerührt. Die Mischung wurde nacheinander mit einer 1 proz. wäßrigen Natriumhydroxid-Lösung und mit Wasser gewaschen und getrocknet. Das Toluol wurde unter vermindertem Druck abdestilliert, wonach 44,9 g 2-Benzylthioethyl-2-[4-(3,5-dichloro-2-pyridyloxy)phenoxy]-propionat als farbloses viskoses Öl erhalten wurden; $n_D^{20}$ = 1,5940.

Nach analogen Verfahren wie in dem Synthese-Beispiel 4 wurden die in der Tabelle 8 aufgeführten Verbindungen der allgemeinen Formel (I) hergestellt.

Tabelle 8

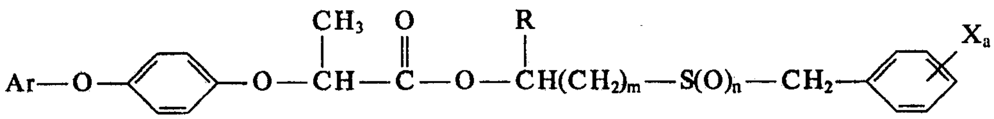

| Verbindung Nr. | Ar | R | m | n | $X_a$ | Physikalische Konstante |
|---|---|---|---|---|---|---|
| 13 | 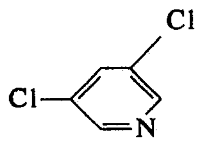 | $-CH_3$ | 1 | 0 | H | $n_D^{20}$ 1.5825 |
| 14 | 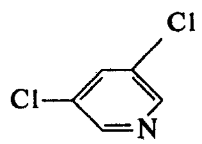 | H | 2 | 0 | H | $n_D^{20}$ 1.5898 |
| 15 | 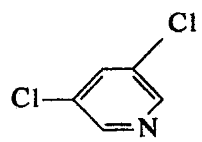 | H | 1 | 0 | 2-F | $n_D^{20}$ 1.5850 |
| 16 | 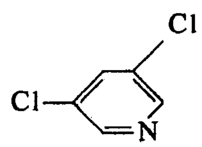 | H | 1 | 0 | 4-Cl | $n_D^{20}$ 1.5972 |
| 17 | 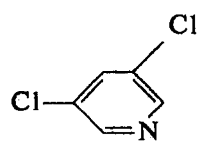 | H | 1 | 0 | $4-CH_3$ | $n_D^{20}$ 1.5920 |
| 18 | 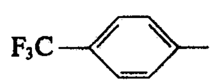 | H | 1 | 0 | $3-NO_2$ | $n_D^{20}$ 1.5630 |
| 19 | 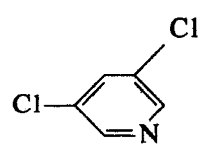 | H | 1 | 0 | $3-NO_2$ | $n_D^{20}$ 1.6037 |
| 20 | 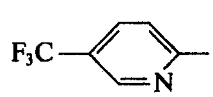 | H | 1 | 0 | $4-OCH_3$ | $n_D^{20}$ 1.5530 |

Synthese-Beispiel 5

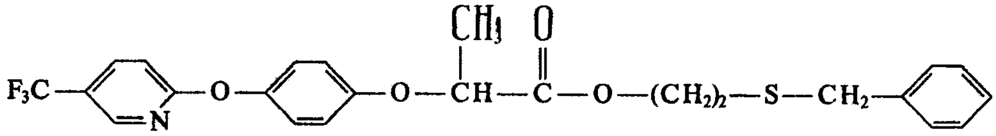

(Verbindung Nr. 21)

33,2 g 2-Benzylthioethyl-2-(4-hydroxyphenoxy)propionat wurden in 100 ml Dimethylsulfoxid gelöst, und 15,2 g Kaliumcarbonat wurden zugesetzt. Unter Rühren wurde die Mischung 1 h auf 90°C erhitzt. Dann wurden bei 80°C bis 90°C 20,0 g 2-Chloro-5-trifluoromethylpyridin hinzugefügt. Nach der Zugabe wurde die Mischung weitere 2 h bei dieser Temperatur gerührt und dann auf Raumtemperatur abgekühlt. Die Mischung wurde in Eiswasser gegossen und mit Chloroform extrahiert. Die Chloroform-Schicht wurde entwässert, und das Chloroform wurde abdestilliert, wonach 36,3 g 2-Benzylthioethyl-2-[4-(5-trifluoromethyl-2-pyridyloxy)phenoxy]propionat als blaßgelbes viskoses Öl erhalten wurde; $n_D^{20} = 1{,}5505$.

Nach analogen Verfahren wie in dem Synthese-Beispiel 5 wurden die in der Tabelle 9 aufgeführten Verbindungen der allgemeinen Formel (I) hergestellt.

Tabelle 9

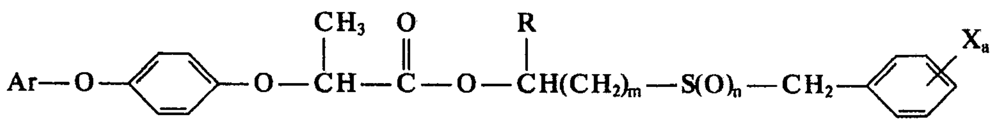

| Verbindung Nr. | Ar | R | m | n | $X_a$ | Physikalische Konstante |
|---|---|---|---|---|---|---|
| 22 | 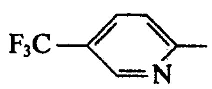 | H | 1 | 0 | 2-F | $n_D^{20}$ 1.5430 |
| 23 | 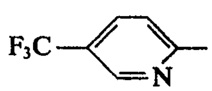 | H | 1 | 0 | 4-Cl | $n_D^{20}$ 1.5566 |
| 24 | 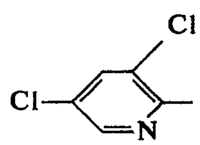 | H | 1 | 0 | 2,4-$Cl_2$ | $n_D^{20}$ 1.6025 |

Synthese-Beispiel 6

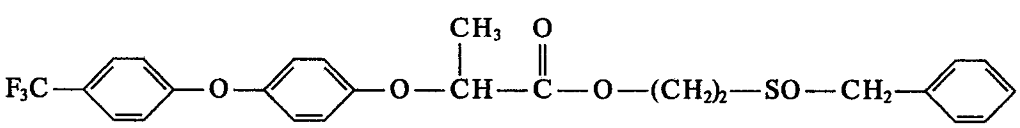

(Verbindung Nr. 25)

47,6 g 2-Benzylthioethyl-2-[4-(4-trifluoromethylphenoxy)phenoxy]propionat wurden in 200 ml Essigsäure gelöst, und 10,7 g 35-proz. wäßriges Hydrogenperoxid wurde bei 20°C bis 30°C tropfenweise zu der Lösung hinzugefügt. Da die Reaktion exotherm war, wurde sie unter Kühlung durchgeführt. Nach der Zugabe wurde die Mischung weitere 4 h bei der gleichen Temperatur gerührt.

Die Reaktionsmischung wurde in Wasser gegossen und mit Chloroform extrahiert. Die Chloroform-Schicht wurde getrocknet, und das Chloroform wurde danach unter vermindertem Druck abdestilliert, wonach 46,7 g 2-Benzylsulfinylethyl-2-[4-(4-trifluoromethylphenoxy)phenoxy]propionat als viskoses Öl erhalten wurden. Beim Stehenlassen erstarrte die Verbindung allmählich und zeigte einen Schmelzpunkt von 100°C bis 112°C.

**Patentansprüche**

1. Substituierte Phenoxypropionate der nachstehenden allgemeinen Formel

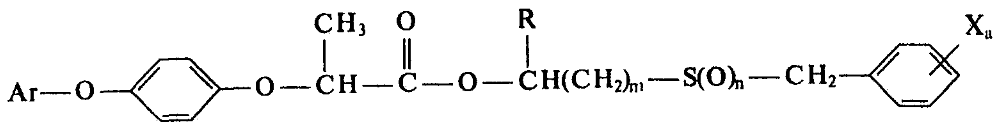

(I)

in der

Ar die Gruppe 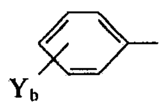 oder die Gruppe 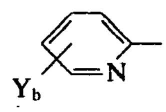 bezeichnet,

in der Y für ein Halogen-Atom oder eine Trifluoromethyl-Gruppe und b für 1 oder 2 stehen,

R ein Wasserstoff-Atom oder eine Methyl-Gruppe bezeichnet,

X ein Wasserstoff-Atom, ein Halogen-Atom, eine Nitro-Gruppe, eine niedere Alkyl-Gruppe oder eine niedere Alkoxy-Gruppe bezeichnet,

a und m jeweils für 1 oder 2 stehen und

n für 0, 1 oder 2 steht.

2. Substituierte Phenoxypropionate gemäß Anspruch 1, gekennzeichnet durch die Formeln

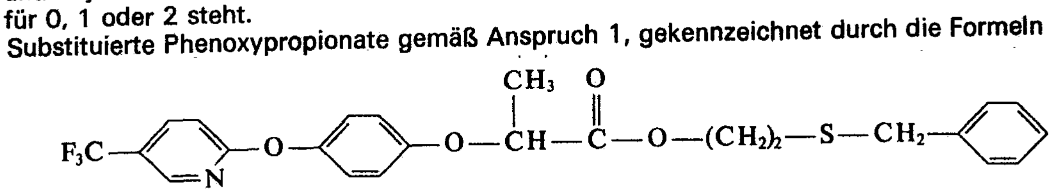

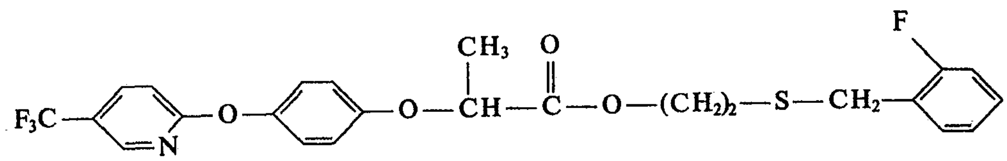

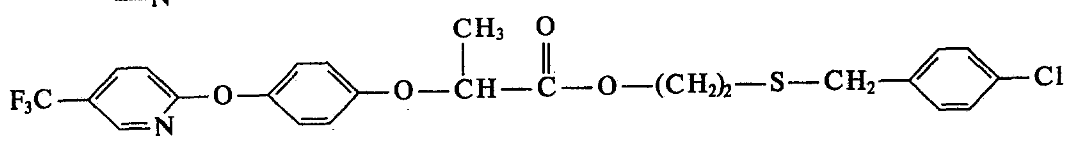

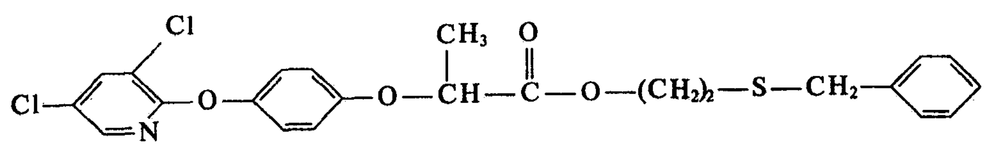

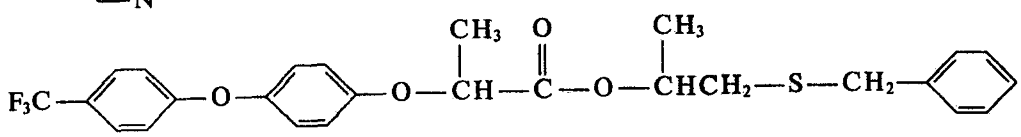

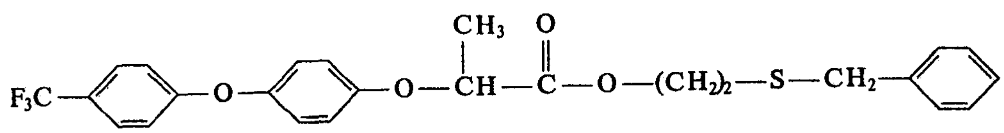

und

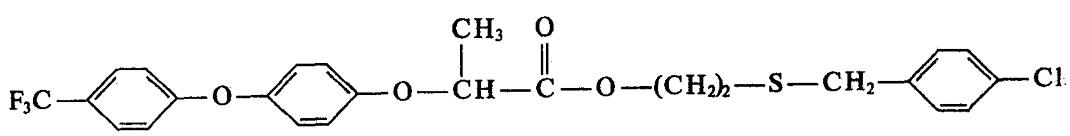

3. Verfahren zur Herstellung von substituierten Phenoxypropionaten der nachstehenden allgemeinen Formel

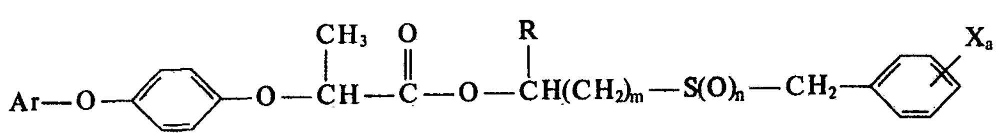

(I)

in der

Ar die Gruppe 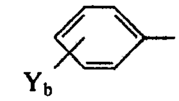 oder die Gruppe 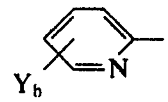 bezeichnet,

in der Y für ein Halogen-Atom oder eine Trifluoromethyl-Gruppe und b für 1 oder 2 stehen,
R ein Wasserstoff-Atom oder eine Methyl-Gruppe bezeichnet,
X ein Wasserstoff-Atom, ein Halogen-Atom, eine Nitro-Gruppe, eine niedere Alkyl-Gruppe oder eine niedere Alkoxy-Gruppe bezeichnet,
a und m jeweils für 1 oder 2 stehen und
n für 0, 1 oder 2 steht,

dadurch gekennzeichnet, daß man

a) Verbindungen der allgemeinen Formel

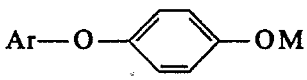

(II)

in der
Ar die im Vorstehenden angegebene Bedeutung hat und
M ein Wasserstoff-Atom oder ein Alkylimetall-Atom bezeichnet,
mit einer Verbindung der allgemeinen Formel

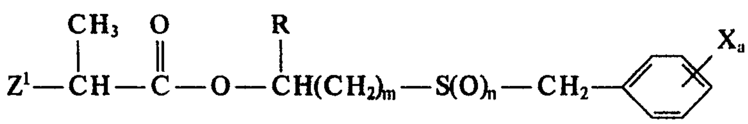

(III)

in der
R, X, a, m und n die im Vorstehenden angegebenen Bedeutungen haben und
$Z^1$ für ein Halogen-Atom steht,
umsetzt,
oder
b) Verbindungen der allgemeinen Formel

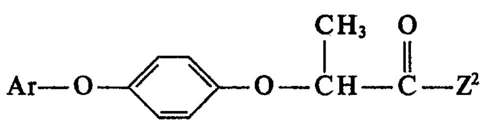

(IV)

in der
Ar die im Vorstehenden angegebene Bedeutung hat und
$Z^2$ für eine Hydroxyl-Gruppe oder ein Halogen-Atom steht,
mit einer Verbindung der allgemeinen Formel

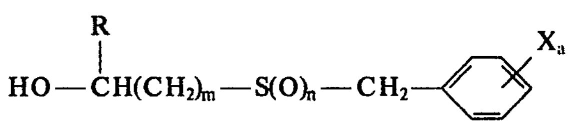

(V)

in welcher
R, X, a, m und n die oben angegebene Bedeutung haben,
umsetzt,
oder
c) Verbindungen der allgemeinen Formel

$$Ar—Z^1 \qquad (VI)$$

in der
Ar die im Vorstehenden angegebene Bedeutung hat und
$Z^1$ ein Halogen-Atom bezeichnet,
mit einer Verbindung der allgemeinen Formel

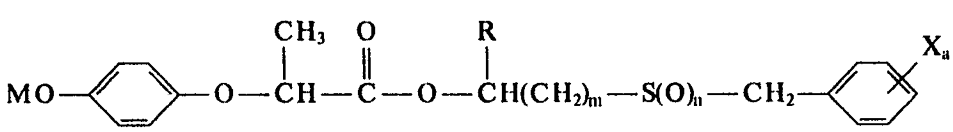

(VII)

in der
R, X, a, m und n die im Vorstehenden angebebenen Bedeutungen haben und
M ein Wasserstoff-Atom oder ein Alkalimetall-Atom bezeichnet,
umsetzt,
oder

d) Verbindungen der allgemeinen Formel

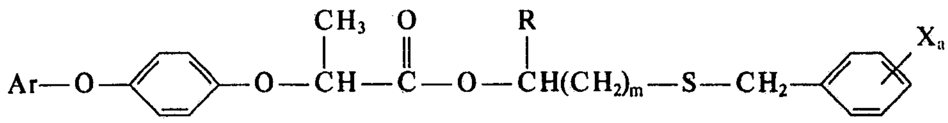

(I-i)

in der
Ar, R, X, a und m die oben angegebene Bedeutung haben,
mit Wasserstoffperoxid umsetzt.

4. Herbizide Mittel, gekennzeichnet durch einen Gehalt an mindestens einem substituierten Phenoxypropionat der Formel (I).

5. Verwendung von substituierten Phenoxypropionaten der Formel (I) zur Bekämpfung von Unkräutern.

6. 2-Halogenpropionate der allgemeinen Formel

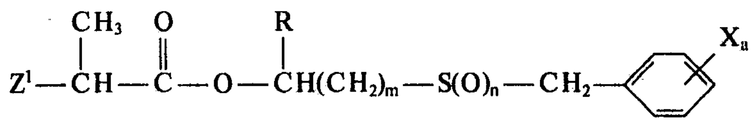

(III)

in der

R ein Wasserstoff-Atom oder eine Methyl-Gruppe bezeichnet,
X ein Wasserstoff-Atom, ein Halogen-Atom, eine Nitro-Gruppe, eine niedere Alkyl-Gruppe oder eine niedere Alkoxy-Gruppe bezeichnet,
a und m jeweils für 1 oder 2 stehen,
n für 0, 1 oder 2 steht und
$Z^1$ ein Halogen-Atom bezeichnet.

7. Verfahren zur Herstellung von 2-Halogenpropionaten der allgemeinen Formel

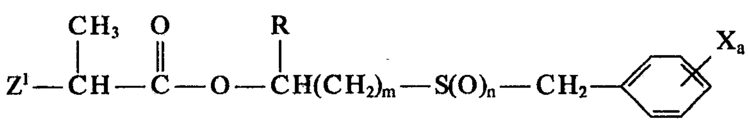

(III)

in der

R ein Wasserstoff-Atom oder eine Methyl-Gruppe bezeichnet,
X ein Wasserstoff-Atom, ein Halogen-Atom, eine Nitro-Gruppe, eine niedere Alkyl-Gruppe oder eine niedere Alkoxy-Gruppe bezeichnet,
a und m jeweils für 1 oder 2 stehen,
n für 0, 1 oder 2 steht und
$Z^1$ ein Halogen-Atom bezeichnet,

dadurch gekennzeichnet, daß man Verbindungen der allgemeinen Formel

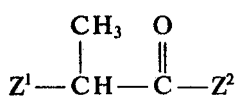

(VIII)

in der

$Z^1$ die im vorstehenden angegebene Bedeutung hat und
$Z^2$ für eine Hydroxyl-Gruppe oder ein Halogen-Atom steht,

mit einer Verbindung der allgemeinen Formel

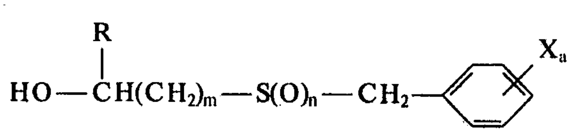

(V)

in der
R, X, a, m und n die im Vorstehenden angegebenen Bedeutungen haben, umsetzt.

8. 2-(4-Hydroxyphenoxy)propionate der allgemeinen Formel

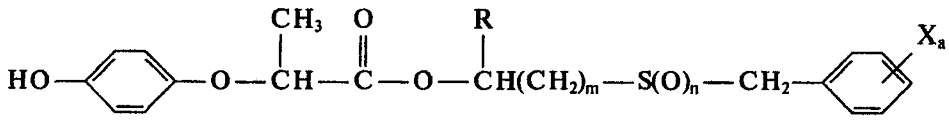

(VII-i)

in der

R ein Wasserstoff-Atom oder eine Methyl-Gruppe bezeichnet,
X ein Wasserstoff-Atom, ein Halogen-Atom, eine Nitro-Gruppe, eine niedere Alkyl-Gruppe oder eine niedere Alkoxy-Gruppe bezeichnet,
a und m jeweils für 1 oder 2 stehen und
n für 0, 1 oder 2 steht.

9. Verfahren zur Herstellung von 2-(4-Hydroxyphenoxy)-propionaten der allgemeinen Formel

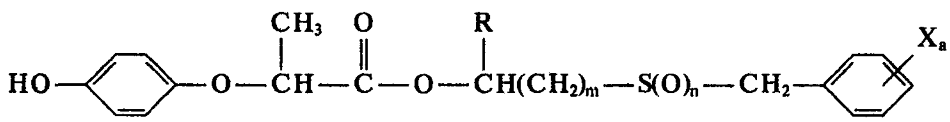

(VII-i)

in der

R ein Wasserstoff-Atom oder eine Methyl-Gruppe bezeichnet,
X ein Wasserstoff-Atom, ein Halogen-Atom, eine Nitro-Gruppe, eine niedere Alkyl-Gruppe oder eine niedere Alkoxy-Gruppe bezeichnet,
a und m jeweils für 1 oder 2 stehen und
n für 0, 1 oder 2 steht,

dadurch gekennzeichnet, daß man Hydrochinon der Formel

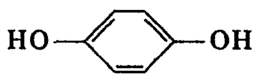

mit einem 2-Halogenopropionat der allgemeinen Formel

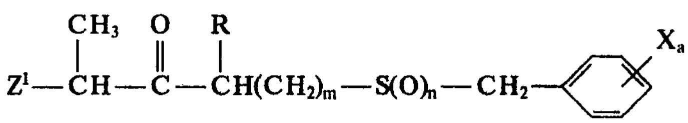

(III)

in der

R, X, a, m und n die im Vorstehenden angegebenen Bedeutungen haben und
$Z^1$ ein Halogen-Atom bezeichnet,

umsetzt.

**Claims**

1. Substituted phenoxypropionates of the following general formula

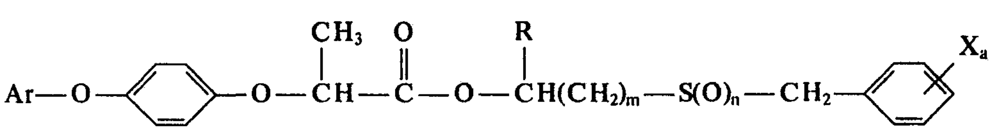

(I)

in which

Ar denotes the group 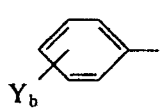 or the group 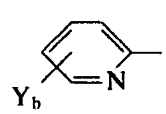

in which Y represents a halogen atom or a trifluoromethyl group and b represents 1 or 2,

R denotes a hydrogen atom or a methyl group,
X denotes a hydrogen atom, a halogen atom, a nitro group, a lower alkyl group or a lower alkoxy group,
a and m each represent 1 or 2 and
n represents 0, 1 or 2.

2. Substituted phenoxypropionates according to Claim 1, characterised by the formulae

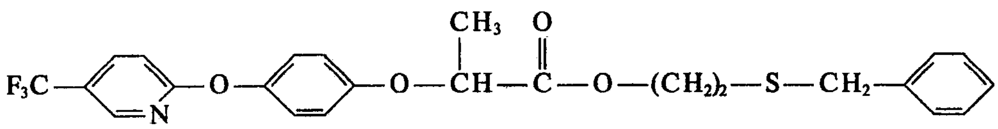

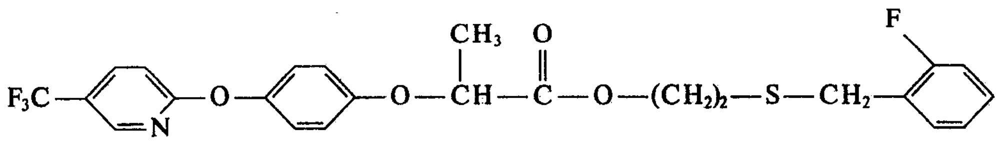

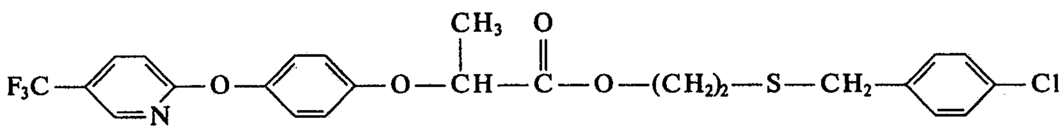

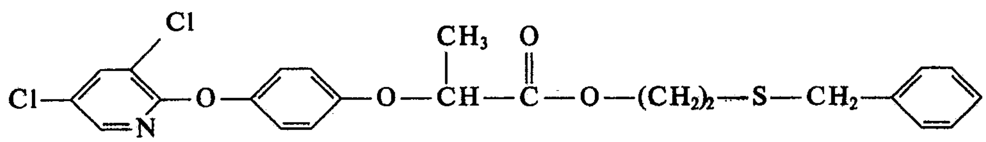

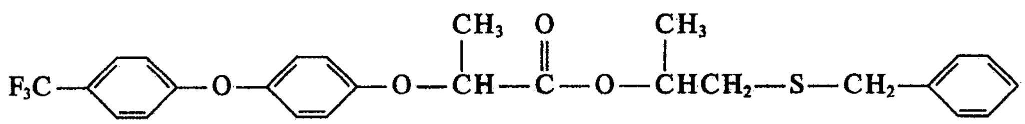

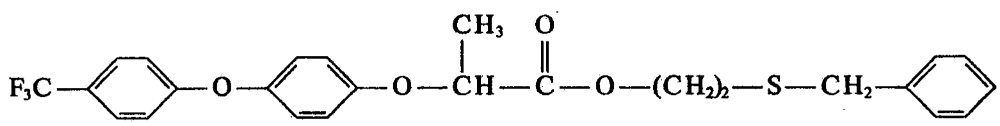

and

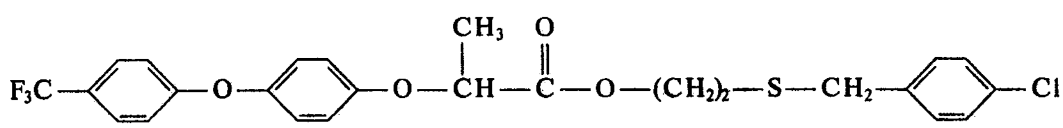

3. Process for the preparation of substituted phenoxypropionates of the following general formula

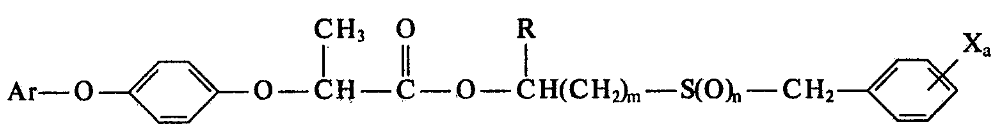 (I)

in which

Ar denotes the group 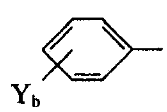 or the group 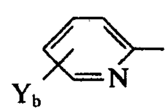

in which Y represents a halogen atom or a trifluoromethyl group and b represents 1 or 2,
R denotes a hydrogen atom or a methyl group,
X denotes a hydrogen atom, a halogen atom, a nitro group, a lower alkyl group or a lower alkoxy group,
a and m each represent 1 or 2 and
n represents 0, 1 or 2,

characterised in that

a) compounds of the general formula

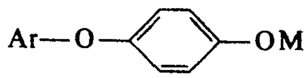

(II)

in which
Ar has the meaning given above and
M denotes a hydrogen atom or an alkali metal atom
are reacted with a compound of the general formula

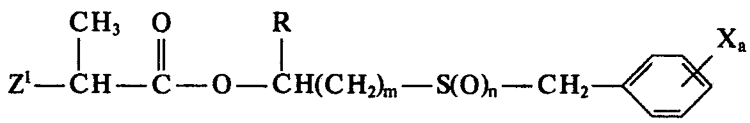

(III)

in which
R, X, a, m and n have the meanings given above and
$Z^1$ represents a halogen atom
or

b) compounds of the general formula

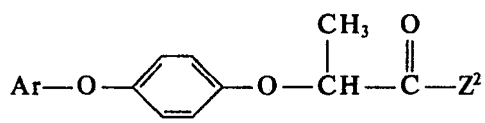

(IV)

in which
Ar has the meaning given above and
$Z^2$ represents a hydroxyl group or a halogen atom
are reacted with a compound of the general formula

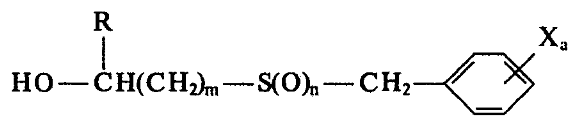

(V)

in which
R, X, a, m und n have the abovementioned meaning,
or

c) compounds of the general formula

$Ar—Z^1$ (VI)

in which
Ar has the meaning given above and
$Z^1$ denotes a halogen atom
are reacted with a compound of the general formula

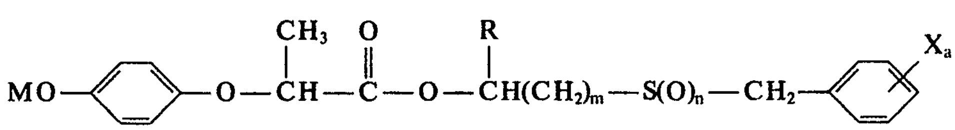

(VII)

in which
R, X, a, m und n have the meanings given above and
M denotes a hydrogen atom or an alkali metal atom
or

d) compounds of the general formula

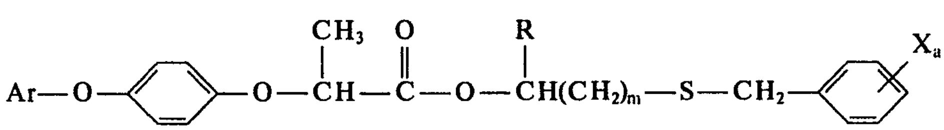

(I-i)

in which
Ar, R, X, a and m have the abovementioned meaning
are reacted with hydrogen peroxide.

4. Herbicidal agent, characterised in that it contains at least one substituted phenoxypropionate of the formula (I).

5. Use of substituted phenoxypropionates of the formula (I) for combating weeds.

6. 2-Halogenopropionates of the general formula

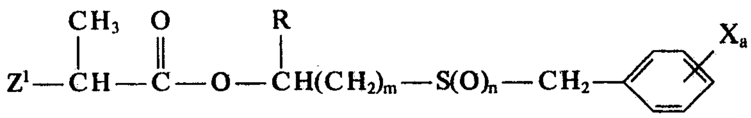

(III)

in which

R denotes a hydrogen atom or a methyl group,
X denotes a hydrogen atom, a halogen atom, a nitro group, a lower alkyl group or a lower alkoxy group,
a and m each represent 1 or 2,
n represents 0, 1 or 2 and
$Z^1$ denotes a halogen atom.

7. Process for the preparation of 2-halogenopropionates of the general formula

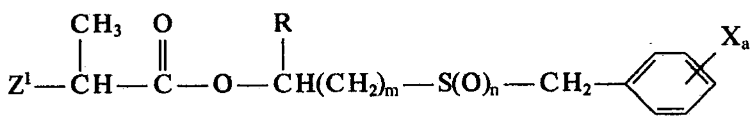

(III)

in which

R denotes a hydrogen atom or a methyl group,
X denotes a hydrogen atom, a halogen atom, a nitro group, a lower alkyl group or a lower alkoxy group,
a and m each represent 1 or 2,
n represents 0, 1 or 2 and
$Z^1$ denotes a halogen atom.

characterised in that compounds of the general formula

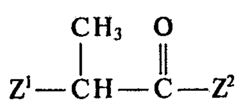

(VIII)

in which

$Z^1$ has the meaning given above and
$Z^2$ represents a hydroxyl group or a halogen atom

are reacted with a compound of the general formula

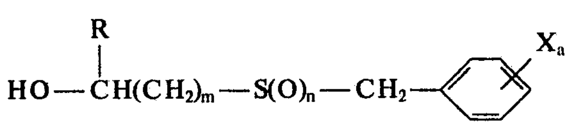

(V)

in which
R, X, a, m and n have the meanings given above.

8. 2-(4-Hydroxyphenoxy)-propionates of the general formula

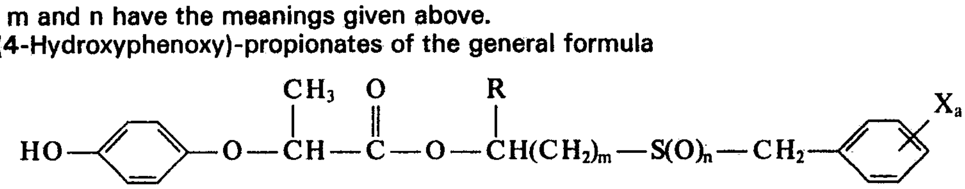

(VII-i)

in which

R denotes a hydrogen atom or a methyl group,
X denotes a hydrogen atom, a halogen atom, a nitro group, a lower alkyl group or a lower alkoxy group,
a and m each represent 1 or 2 and
n represents 0, 1 or 2.

9. Process for the preparation of 2-(4-hydroxyphenoxy)-propionates of the general formula

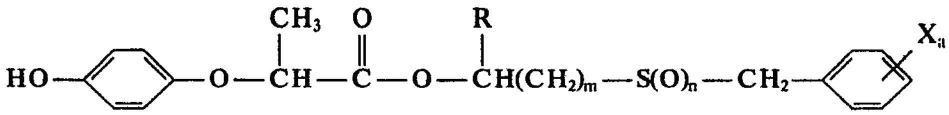 (VII-i)

in which

R denotes a hydrogen atom or a methyl group,
X denotes a hydrogen atom, a halogen atom, a nitro group, a lower alkyl group or a lower alkoxy group,
a and m each represent 1 or 2 and
n represents 0, 1 or 2,

characterised in that hydroquinone of the formula

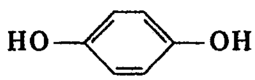

is reacted with a 2-halogenopropionate of the general formula

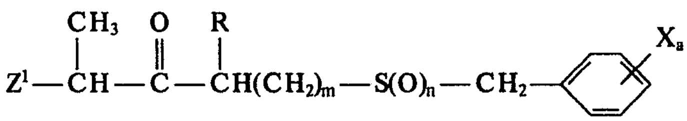 (III)

in which

R, X, a, m and n have the meanings given above and
$Z^1$ denotes a halogen atom.

**Revendications**

1. Phénoxypropionates substitués de formule générale suivante:

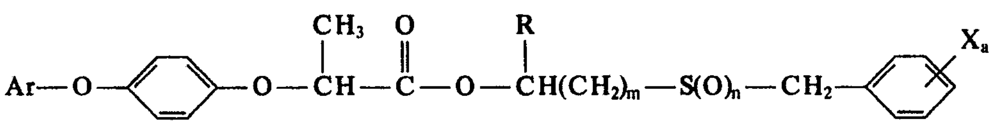 (I)

dans laquelle

Ar représente le groupe 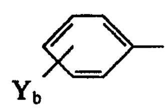 ou le groupe 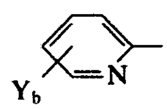

où Y représente un atome d'halogène ou un groupe trifluorométhyle et b a la valeur 1 ou 2,
R est un atome d'hydrogène ou un groupe méthyle,
X est un atome d'hydrogène, un atome d'halogène, un groupe nitro, un groupe alkyle inférieur ou un groupe alkoxy inférieur,
a et m ont chacun la valeur 1 ou 2 et
n a la valeur 0, 1 ou 2.

2. Phénoxypropionates substitués suivant la revendication 1, caractérisés par les formules

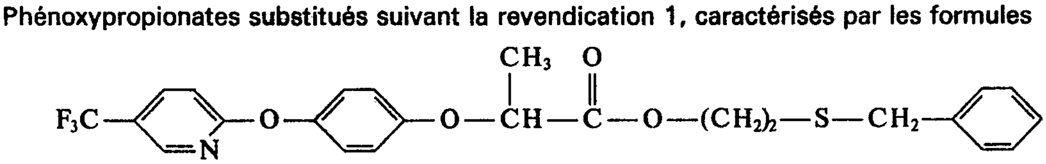

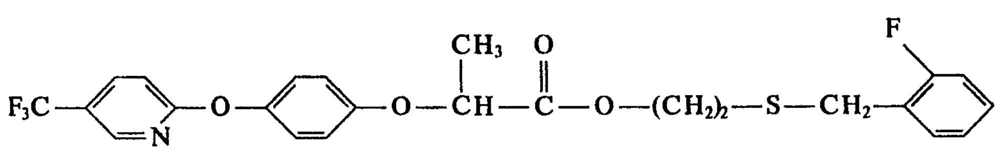

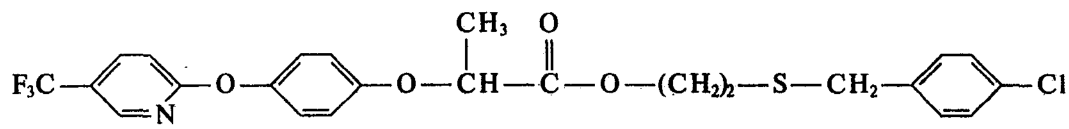

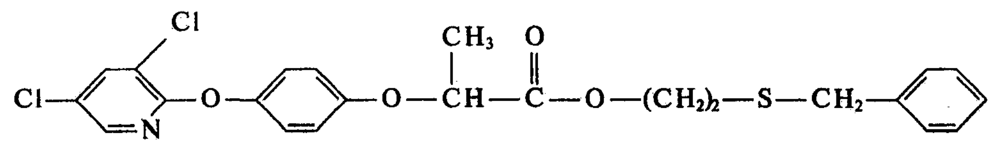

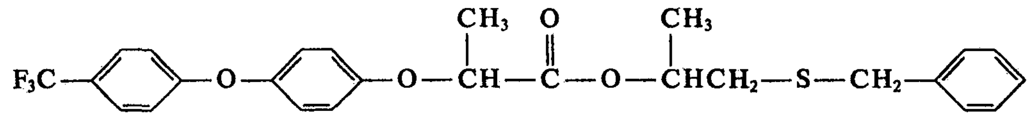

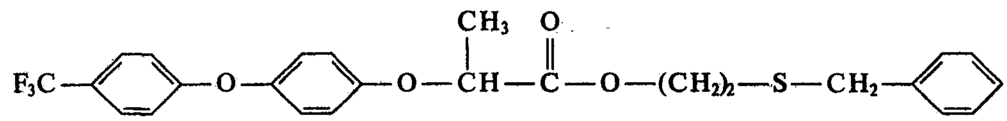

et

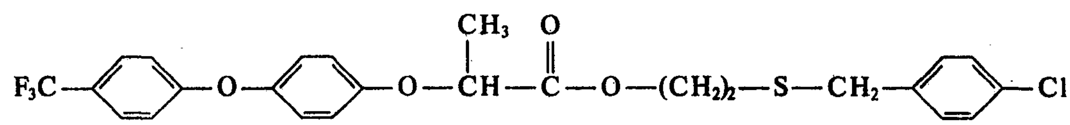

3. Procédé de production de phénoxypropionates substitués de la formule générale suivante

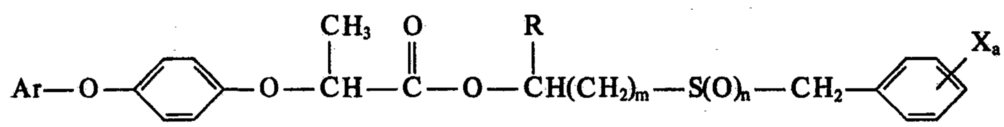 (I)

dans laquelle

Ar représente le groupe 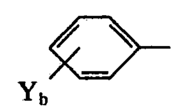 ou le groupe 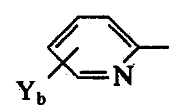

où Y représente un atome d'halogène ou un groupe trifluorométhyle et b a la valeur 1 ou 2,

R est un atome d'hydrogène ou un groupe méthyle,

X est un atome d'hydrogène, un atome d'halogène, un groupe nitro, un groupe alkyle inférieur ou un groupe alkoxy inférieur,

a et m ont chacun la valeur 1 ou 2 et

n a la valeur 0, 1 ou 2,

caractérisé en ce qu'on fait réagir

a) des composés de formule générale

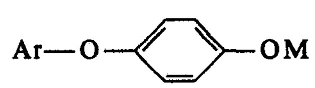 (II)

dans laquelle

Ar a la définition indiquée ci-dessus et

M est un atome d'hydrogène ou un atome de métal alcalin,

avec un composé de formule générale

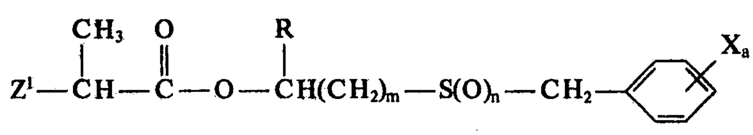 (III)

dans laquelle

R, X, a, m et n ont les définitions indiquées ci-dessus et

$Z^1$ est un atome d'halogène,

ou

b) des composés de formule générale

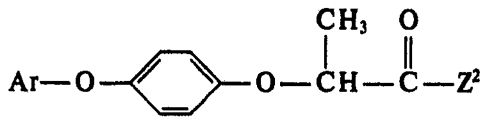

(IV)

dans laquelle
Ar a la définition indiquée ci-dessus et
$Z^1$ est un groupe hydroxyle ou un atome d'halogène,
avec un composé de formule générale

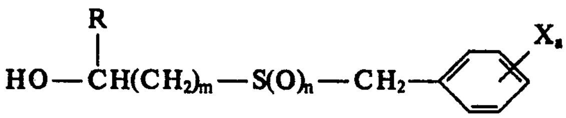

(V)

dans laquelle
R, X, a, m et n ont la définition indiquée ci-dessus,
ou

c) des composés de formule générale

$Ar—Z^1$ (VI)

dans laquelle
Ar a la définition indiquée ci-dessus et
$Z^1$ représente un atome d'halogène,
avec un composé de formule générale

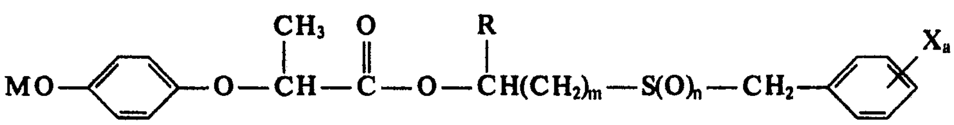

(VII)

dans laquelle
R, X, a, m et n ont les définitions indiquées ci-dessus et
M est un atome d'hydrogène ou un atome de métal alcalin,
ou

d) des composés de formule générale

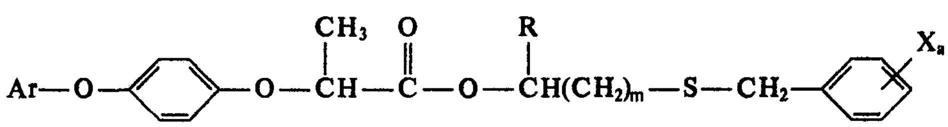

(I-i)

dans laquelle
Ar, R, X, a et m ont la définition indiquée ci-dessus,
avec le peroxyde d'hydrogène.

4. Compositions herbicides, caractérisées par une teneur en au moins un phénoxypropionate substitué de formule (I).

5. Utilisation de phénoxypropionates substitués de formule (I) pour combattre des mauvaises herbes.

6. 2-halogénopropionates de formule générale

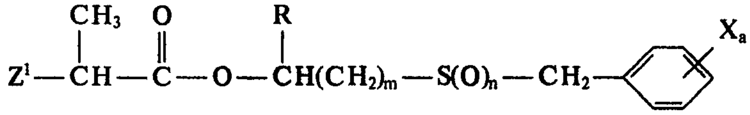

(III)

dans laquelle

R est un atome d'hydrogène ou un groupe méthyle,
X est un atome d'hydrogène, un atome d'halogène, un groupe nitro, un groupe alkyle inférieur ou un groupe alkoxy inférieur,
a et m ont chacun la valeur 1 ou 2,
n a la valeur 0, 1 ou 2 et
$Z^1$ est un atome d'halogène.

7. Procédé de production de 2-halogénopropionates de formule générale

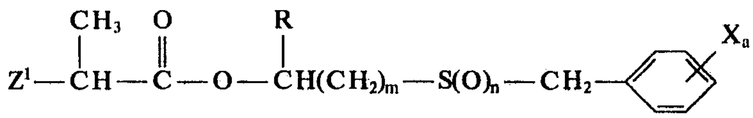

(III)

dans laquelle

R est un atome d'hydrogène ou un groupe méthyle,
X est un atome d'hydrogène, un atome d'halogène, un groupe nitro, un groupe alkyle inférieur ou un groupe alkoxy inférieur,
a et m ont chacun la valeur 1 ou 2,
n a la valeur 0, 1 ou 2 et
$Z^1$ est un atome d'halogène,

caractérisé en ce qu'on fait réagir des composés de formule générale

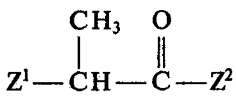

(VIII)

dans laquelle

$Z^1$ a la définition indiquée ci-dessus et
$Z^2$ représente un groupe hydroxyle ou un atome d'halogène,

avec un composé de formule générale

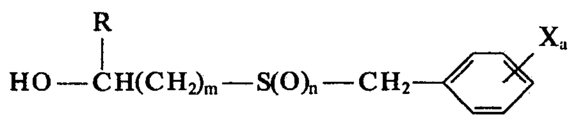

(V)

dans laquelle
R, X, a, m et n ont les définitions indiquées ci-dessus.

8. 2-(4-hydroxyphénoxy)-propionates de formule générale

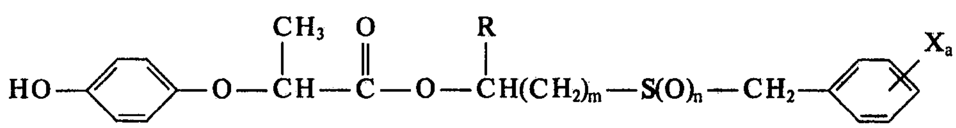

(VII-i)

dans laquelle

R est un atome d'hydrogène ou un groupe méthyle,
X représente un atome d'hydrogène, un atome d'halogène, un groupe nitro, un groupe alkyle inférieur ou un groupe alkoxy inférieur,
a et m ont chacun la valeur 1 or 2 et
n est égal à 0, 1 ou 2.

9. Procédé de production de 2-(4-hydroxyphénoxy)-propionates de formule générale

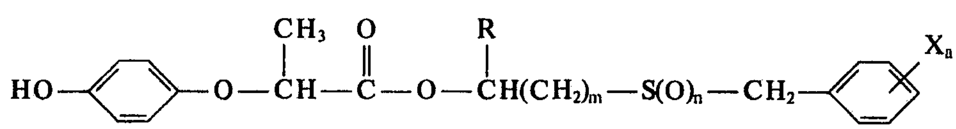

(VII-i)

dans laquelle

R est un atome d'hydrogène ou un groupe méthyle,
X représente un atome d'hydrogène, un atom d'halogène, un groupe nitro, un groupe alkyle inférieur ou un groupe alkoxy inférieur,
a et m ont chacun la valeur 1 ou 2 et
n est égal à 0, 1 ou 2,

caractérisé en ce qu'on fait réagir une hydroquinone de formule

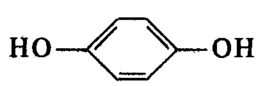

avec un 2-halogénopropionate de formule générale

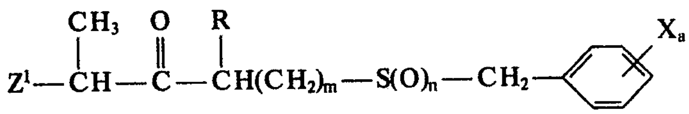

(III)

dans laquelle

R, X, a, m et n ont les définitions indiquées ci-dessus et
$Z^1$ est un atome d'halogène.